# EUROPEAN PATENT APPLICATION

(11) **EP 4 759 780 A1**
(43) Date of publication of application: **17.06.2026**
(21) Application number: 24219584.0
(22) Date of filing: 12.12.2024
(51) Int. Cl.: C03C 3/097, A61K 6/833, C03C 4/00, C03C 10/00

(54) **LITHIUM ALUMOSILICATE GLASS-CERAMIC AND DENTAL BODY COMPRISING THE SAME**

(71) Applicant: Ivoclar Vivadent AG, 9494 Schaan (LI)
(72) Inventor: RITZBERGER, Christian, 9742 Grabs (CH); DITTMER, Marc, 6800 Feldkirch (AT); RAMPF, Markus, 7212 Seewis-Dorf (CH)
(74) Representative: Maiwald GmbH

(57) **Abstract**

The present invention relates to a glass or a glass-ceramic for preparing a lithium alumosilicate glass-ceramic comprising a keatite solid solution crystal phase, wherein the glass or the glass-ceramic comprises 60 to 78 wt.% of SiO₂, 8.5 to 24 wt.% of Li₂O, 2.0 to 15 wt.% of Al₂O₃, and a nucleating agent. The present invention further relates to a lithium alumosilicate glass-ceramic for a dental body, wherein the lithium alumosilicate glass-ceramic comprises 60 to 78 wt.% of SiO₂, 8.5 to 24 wt.% of Li₂O, 2.0 to 15 wt.% of Al₂O₃, and a nucleating agent, and wherein the lithium alumosilicate glass-ceramic comprises a keatite solid solution crystal phase.

## Description

### TECHNICAL FIELD

The present invention relates to a glass or glass-ceramic for preparing a lithium alumosilicate glass-ceramic comprising a keatite solid solution crystal phase. The present invention further relates to a lithium alumosilicate glass-ceramic comprising a keatite solid solution crystal phase and to a dental body comprising a glass-ceramic body comprising the lithium alumosilicate glass-ceramic. Furthermore, the present invention relates to processes for preparing said materials.

### BACKGROUND OF INVENTION

Glass-ceramics are widely used in different technical areas. In the dental field, glass-ceramics play a significant role as base materials for dental bodies, and particularly for dental restorations. Typically, the glass-ceramics are provided in form of dental blanks such as dental mill blanks or dental press blanks. A glass-ceramic dental mill blank can be machined, usually using a CAD/CAM process, to a desired shape of the dental restoration. A glass-ceramic dental press blank can be hot pressed in a mold having a desired shape of the dental restoration. Furthermore, 3D printing processes have recently gained significance in which a dental body is formed by printing a glass precursor or a glass-ceramic precursor which may subsequently be converted into a desired glass-ceramic.

WO 2012/143137 A1 describes a glass-ceramic body and its use for a dental restoration.

The appearance of natural teeth varies depending on the age of a person, its lifestyle, its health, etc.. There are nevertheless some optical characteristics that may be seen as universal for most natural human teeth. A natural tooth usually has a color and translucency that changes from the upper incisal or occlusal part of the tooth to its lower dentin part which is closer to the gums. The area between those two parts of the tooth often shows some type of graded change or transition in color and translucency. Ideally, a dental restoration can be prepared in a way that the dental restoration matches remaining, adjacent natural teeth in a person's mouth to provide an aesthetically pleasing appearance. In addition, it is typically desired that the dental restoration has certain mechanical properties, like a specific fracture toughness, to provide sufficient durability and resistance. Furthermore, it is sometimes desirable that the glass-ceramic has a specific thermal expansion, especially when the glass-ceramic is used in form of a veneer or when the glass-ceramic is to be coated with a glazing.

In the prior art, efforts have been made to create different crystal phases in different parts of a glass-ceramic dental material by irradiating different parts of a surface of a glass with a laser. Thereby, the optical properties of the glass-ceramic may be varied across the material. This technology has however the disadvantage that the laser irradiation typically has a limited penetration depth which in turn predominantly results in a heating/cooling of the material which is located closer to the surface. Thereby, the formation of crystal phases may not be uniformly distributed within the glass-ceramic material. Furthermore, tensions may build up in the glass-ceramic material due to the local temperature changes.

There is a continuing need in the art for glass-ceramic materials which are suitable for use in a dental restoration and which can be adjusted to match the natural appearance of a tooth or parts thereof. There is further a need to provide a glass-ceramic material which is suitable for use in a dental restoration and which can be adjusted in its properties throughout its entire bulk material, including a core part of the glass-ceramic material. There is a continuing need in the art for dental bodies, particularly dental restorations or dental blanks for preparing the said dental restorations, which mimic closely the natural appearance of a tooth including its change in color and/or translucency.

### OBJECTS AND SUMMARY OF THE INVENTION

One object of the present invention is to provide a glass or glass-ceramic precursor which can be used to prepare a glass-ceramic for a dental body. One object of the present invention is to provide a glass-ceramic for a dental body which is suitable to provide a dental body or a part thereof with a desirable color, translucency and/or thermal expansion. One object of the present invention is to provide a dental body, and particularly a dental restoration or a dental blank for preparing said dental restoration, which has a highly aesthetic appearance.

One or more of the above objects is solved by the glass or glass-ceramic, the lithium alumosilicate glass-ceramic, the dental body, and the processes and uses according to the aspects and embodiments of the present invention.

In one aspect, the present invention provides a glass or a glass-ceramic for preparing a lithium alumosilicate glass-ceramic comprising a keatite solid solution crystal phase, wherein the glass or the glass-ceramic comprises 60 to 78 wt.% of SiO₂, 8.5 to 24 wt.% of Li₂O, 2.0 to 15 wt.% of Al₂O₃, and a nucleating agent. In one embodiment, the glass or glass-ceramic is a powder.

In one aspect, the present invention provides a lithium alumosilicate glass-ceramic for a dental body, wherein the lithium alumosilicate glass-ceramic comprises 60 to 78 wt.% of SiO₂, 8.5 to 24 wt.% of Li₂O, 2.0 to 15 wt.% of Al₂O₃, and a nucleating agent, and wherein the lithium alumosilicate glass-ceramic comprises a keatite solid solution crystal phase.

The inventors found that a glass or glass-ceramic as provided herein is useful for preparing a lithium alumosilicate glass-ceramic with a keatite solid solution crystal phase. The inventors have further found that the lithium alumosilicate glass-ceramic as provided herein has specific mechanical, optical and/or thermal properties which make it particularly useful for preparing a dental body. For example, the lithium alumosilicate glass-ceramic may be used to provide a specific translucency, coefficient of thermal expansion, and/or fracture toughness to a part, a layer, or a section of a dental body, or to a dental body as a whole. For example, when the lithium alumosilicate glass-ceramic is adjusted to have a high content of the keatite solid solution crystal phase, a glass-ceramic with a comparatively high translucency and/or low coefficient of thermal expansion may be provided. Based thereon, the inventors further found that one type of the lithium alumosilicate glass-ceramic may be used in combination with one or more different types of the lithium alumosilicate glass-ceramic and/or in combination with a different glass-ceramic material (not containing a keatite sold solution crystal phase) in a dental body. Thereby, a dental body may be provided which shows a variation of one or more properties, like color, translucency, and/or thermal expansion.

In one embodiment, the molar ratio of SiO₂ to Li₂O is in a range of 1.50 to 4.22, preferably in a range of 1.80 to 4.00, and more preferably in a range of 2.00 to 3.80. In one embodiment, the weight amount of Al₂O₃ is in a range of 2.0 to 10.0 wt.%.

In one embodiment, the lithium alumosilicate glass-ceramic comprises the keatite solid solution crystal phase in an amount in a range of 2.5 to 50 wt.%, based on the total weight of the lithium alumosilicate glass-ceramic.

In one embodiment, the keatite solid solution crystal phase is the main crystal phase of the lithium alumosilicate glass-ceramic, and optionally the lithium alumosilicate glass-ceramic comprises one or more of Li₃PO₄, Li₂Si₂O₅, quartz s.s., and Li₂SiO₃, as one or more minor crystal phases, optionally in combination with one or more additional minor crystal phases.

In an alternative embodiment, the main crystal phase of the lithium alumosilicate glass-ceramic is a quartz s. s., Li₂Si₂O₅ or Li₂SiO₃, and the keatite solid solution crystal phase is a minor crystal phase.

One embodiment provides a process for preparing a lithium alumosilicate glass-ceramic according to the present invention, the process comprising the steps of:
- providing a powder selected from glass powders, glass-ceramic powders and mixtures thereof;
- preparing a shaped body from the powder; and
- subjecting the shaped body to a heat treatment,
   or
- preparing a solid glass body from a molten glass; and
- subjecting the solid glass body to a heat treatment.

In one aspect, the present invention provides a dental body comprising a glass-ceramic body comprising the lithium alumosilicate glass-ceramic according to the present invention.

In one embodiment, the glass-ceramic body comprises:
a top layer, and
a bottom layer,
wherein the top layer is a layer of one type of the lithium alumosilicate glass-ceramic, and the bottom layer is either a layer of a different type of the lithium alumosilicate glass-ceramic or a layer of a different glass-ceramic material not containing a keatite s.s. crystal phase, and
wherein the top layer has a weight amount of the keatite s.s. crystal phase which is higher than a weight amount of the keatite s.s. crystal phase in the bottom layer.

In one embodiment, the glass-ceramic body further comprises at least one intermediate layer between the top layer and the bottom layer,
wherein the at least one intermediate layer is at least one layer of another different type of the lithium alumosilicate glass-ceramic, and
wherein the top layer, the at least one intermediate layer, and the bottom layer differ in their weight amount of a keatite s.s. crystal phase such that the weight amount of the keatite s.s. crystal phase increases from the bottom layer to the at least one intermediate layer, and from the at least one intermediate layer to the top layer.

In an alternative embodiment, the glass-ceramic body comprises:
a top section,
an intermediate section, and
a bottom section,
wherein the top section is entirely composed of one type of the lithium alumosilicate glass-ceramic, and the bottom section is entirely composed of a different type of the lithium alumosilicate glass-ceramic or of another glass-ceramic material not containing a keatite s.s. crystal phase, and
wherein the weight amount of the lithium alumosilicate glass-ceramic of the top section gradually changes across the intermediate section in a direction to the bottom layer such that a weight amount of the keatite s.s. crystal phase gradually decreases across the intermediate section in a direction to the bottom layer.

In one embodiment, the glass-ceramic body is characterized by a translucency gradient and/or a gradient of a coefficient of thermal expansion.

In one embodiment, the dental body is a dental blank for preparing a dental restoration, or wherein the dental body is a dental restoration.

Further aspects and embodiments of the present invention are disclosed herein in the detailed description provided herein below.

### DEFINITIONS

In the context of the present invention, the following terms have the following meaning:

"Dental body" means a solid, geometrically defined, three-dimensional object of material, like an ingot, block, a disc, or a form of a dental restoration, which is suitable for application in the dental or orthodontic field. A dental body may be, but is not limited to, a dental blank or a dental restoration. A dental blank may be a dental mill blank or a dental press blank.

"Dental mill blank" means a solid, geometrically defined, three-dimensional object of material, like a block or a disc, from which a dental restoration can be machined by, e.g., cutting, milling, grinding, drilling, and the like, typically using a CAD/CAM process.

"Dental press blank" means a solid, geometrically defined, three-dimensional object of material, like a block or an ingot, from which a dental restoration can be formed by hot pressing the blank into a mold having a shape of a dental restoration.

"Dental restoration" as used herein refers to an article that is useful in the dental or orthodontic field for restoring, remodeling, supporting and/or restructuring a tooth or parts thereof or a group of teeth or parts thereof. A dental restoration may be, but is not limited to, a crown, a partial crown, an abutment, an abutment crown, an inlay, an onlay, a veneer, a shell or a bridge.

"Glass-ceramic" means an inorganic, non-metallic solid having a glass phase which surrounds one or more crystal phases. A glass-ceramic material is typically obtained via a controlled nucleation and crystallization of an amorphous base glass. A glass-ceramic material is different from a ceramic material which is typically free of a glass phase. A "glass" means an inorganic, non-metallic solid which is typically hard, brittle and transparent and essentially free of crystalline regions. It may be described as a thermodynamically unstable frozen melt. In one embodiment, the glass-ceramic does not contain a glass-matrix-composite or is not composed of a glass-matrix composite. A "glass-matrix composite" in the meaning of the present disclosure is a material which is obtained by addition of crystalline particles to a glass melt or by attaching (e.g., by sintering) a glass material to a crystalline material.

A "keatite solid solution" (also abbreviated herein as "keatite s.s.") or "keatite s.s. crystal phase" means a tetragonal polymorph of SiOz at room temperature. Si⁴⁺ is partially replaced by Al³⁺ in the tectosilicate framework according the general formula: Li_{X}Al_{X}Si_{1-X}O₂.

A "quartz solid solution" (also abbreviated herein as "quartz s.s.") or "quartz s.s. crystal phase" means a trigonal or hexagonal polymorph of SiOz at room temperature, in which foreign ions are either incorporated into interstitial sites or into lattice sites. These foreign ions may be, but are not limited to, Al³⁺ as well as Mg²⁺, Li⁺ and/or Zn²⁺. For example, Al³⁺ may partially replace Si⁴⁺ and the remaining negative charge may be neutralized by Li⁺. Zn²⁺ and Mg²⁺ may neutralize two negative charges, e.g., which may be formed by replacement of two Si⁴⁺ with two Al³⁺.

A "main crystal phase" refers to the crystal phase of the glass-ceramic which has the highest mass fraction of all the crystal phases being present in the glass-ceramic. The masses of the crystal phases can be determined quantitatively using the Rietveld method, e.g., as described herein in the measuring methods. A "minor crystal phase" refers to a crystal phase of the glass-ceramic which has a fraction in the glass-ceramic which is lower than the weight fraction of the main crystal phase.

"Top section" or "top layer" as used herein refers to an outermost section or layer of a glass-ceramic body of a dental body. When the dental body is a dental blank for preparing a dental restoration, a top section or a top layer is useful for preparing an incisal or occlusal zone, or a part thereof, of the dental restoration. Alternatively, when the dental body is a dental restoration, a top section of a top layer may form an incisal or occlusal zone, or a part thereof, in a dental restoration.

"intermediate section" or "intermediate layer" means a section or a layer that is positioned between the top section/top layer and the bottom section/bottom layer of the glass-ceramic body of a dental body. When the dental body is a dental blank for preparing a dental restoration, an intermediate section or an intermediate layer is useful for preparing a transition zone, or a part thereof, of the dental restoration. Alternatively, when the dental body is a dental restoration, an intermediate section or an intermediate layer may form transition zone, or a part thereof, in a dental restoration.

"Bottom section" or "bottom layer" means an outermost section or layer of a glass-ceramic body of a dental body, wherein the section or layer is located on an opposite side of the glass-ceramic body with respect to the top section. When the dental body is a dental blank for preparing a dental restoration, a bottom section or bottom layer is useful for preparing a dentin zone, or a part thereof, of the dental restoration. Alternatively, when the dental body is a dental restoration, a bottom section or bottom layer may form dentin zone, or a part thereof, in a dental restoration.

The terms "top section", "intermediate section", "bottom section", "top layer", "intermediate layer", and "bottom layer" as used herein are not to be construed in that the glass-ceramic body or dental body necessarily has to be positioned (or used) in a specific manner or direction.

A "layer" means a discrete layer of a glass-ceramic body. Discrete layers in a glass-ceramic body can be determined by microscopy, like single electron microscopy (SEM).

A "gradient" as used herein means that a property of a glass-ceramic body (e.g., a contrast ratio, a coefficient of thermal expansion (CTE), and the like) increases or decreases (e.g., gradually or in a stepwise manner) in a direction of the glass-ceramic body.

Where the term "comprising" is used herein, it does not exclude that further non-specified elements are present. Where the term "essentially consisting of" is used herein, it does not exclude that further non-specified elements are present that are not materially affecting the essential characteristics of the defined subject-matter. For the purposes of the present invention, the terms "essentially consisting of" and "consisting of" are considered to be specific embodiments of the term "comprising of". Whenever the terms "including" or "having" are used, these terms are meant to be equivalent to "comprising" as defined above.

The term "obtained" does not necessarily mean to indicate that, e.g., an embodiment must be obtained by, e.g., a sequence of steps following the term "obtained" even though such a limited understanding is always included by the term "obtained" as a preferred embodiment.

In the following, the aspects and embodiments of the present invention are described in more detail.

### DETAILED DESCRIPTION

### I. Glass or glass ceramic for preparing a lithium alumosilicate glass-ceramic with a keatite s.s. crystal phase

In one aspect of the present invention, a glass or glass ceramic for preparing a lithium alumosilicate glass-ceramic comprising a keatite s.s. crystal phase is provided. The glass or glass ceramic comprises
60 to 78 wt.% of SiO₂,
8.5 to 24 wt.% of Li₂O,
2.0 to 15 wt.% of Al₂O₃, and
a nucleating agent.

The glass or glass ceramic is suitable for preparing a lithium alumosilicate glass-ceramic comprising a keatite s.s. crystal phase. Accordingly, the glass or glass-ceramic has a composition which is suitable to form a keatite s.s. crystal phase. In particular, the glass or glass-ceramic has a composition which is suitable to form a keatite s.s. crystal phase when subjected to a heat treating, for example, at a (maximum) temperature in a range of 750 to 1050°C, like in a range of 800 to 1000°C, like in a range of 850 to 1000°C, or in a range of 880 to 980°C. A formation of a keatite s.s. crystal phase by heat treating is described in more detail herein below, e.g., in the section II.4.3.1.

### 1. Chemical composition

The glass or glass ceramic comprises
60 to 78 wt.% of SiO₂,
8.5 to 24 wt.% of Li₂O,
2.0 to 15 wt.% of Al₂O₃, and
a nucleating agent.

All weight amounts described in the present section are referenced to the total weight of the glass or glass-ceramic, unless explicitly stated otherwise. The sum of the weight amounts of the components is selected such that a total amount of 100 wt.% is not exceeded.

The glass or glass ceramic comprises 60 to 78 wt.% of SiO₂, like an amount in a range of 62 to 78 wt.%. For example, the glass or glass ceramic may comprise 62 to 69 wt.% of SiOz. The glass or glass ceramic may comprise 70 to 78 wt.% of SiOz.

The glass or glass-ceramic comprises 8.5 to 24 wt.% of Li₂O. For example, the glass or glass-ceramic may comprise 8.5 to 22 wt.% of Li₂O. The glass or glass ceramic may comprise 10 to 20 wt.% of Li₂O.

The glass or glass-ceramic comprises 2.0 to 15 wt.% of Al₂O₃. In many embodiments, the glass or glass ceramic comprises 2.0 to 10.0 wt.%, like in a range of 2.0 to 9.0 wt.%, 2.0 to 8.0 wt.%, 2.0 to 7.0 wt.%, or 2.0 to 6.0 wt.%, of Al₂O₃. It is however also possible that the glass or glass ceramic comprises 10 to 15 wt.% of Al₂O₃.

The molar ratio of SiO₂ to Li₂O may be in a range of 1.50 to 4.22, preferably in a range of 1.80 to 4.00, and more preferably in a range of 2.00 to 3.80.

The ratio of the weight amount of Li₂O to the weight amount of Al₂O₃ may be in a range of 0.5 to 5.7. In one embodiment, the ratio of the weight amount of Li₂O to weight amount of Al₂O₃ is in a range of 0.5 to 0.9. In one embodiment, the ratio of the weight amount of Li₂O to weight amount of Al₂O₃ is in a range of 1.0 to 1.5. In one embodiment, the ratio of the weight amount of Li₂O to weight amount of Al₂O₃ is in a range of 1.6 to 5.7.

In one embodiment, the weight ratio of Li₂O to Al₂O₃ is in a range of 1.6 to 5.7, and the glass or glass-ceramic comprises a weight amount of one valent metal oxides other than Li₂O of less than 2.0 wt.%, less than 1.0 wt.%, or less than 0.5 wt.%. In one embodiment, the weight ratio of Li₂O to Al₂O₃ is in a range of 1.6 to 5.7, and the glass or glass-ceramic is essentially free of (or is free of) one valent metal oxides other than Li₂O. A ratio of the weight amount of Li₂O to the weight amount of Al₂O₃ in a range of 1.6 to 5.7, optionally in combination with a particularly low amount (or absence) of other one valent metal oxides, can provide desirable mechanical properties (e.g., a particularly good fracture toughness) for a lithium alumosilicate glass-ceramic which is obtainable from the glass or glass-ceramic.

In one embodiment, the weight ratio of the Li₂O to Al₂O₃ is in a range of 1.6 to 5.7, and the weight amount of Al₂O₃ is in a range of 2.0 to 10.0 wt.%, like in a range of 2.0 to 9.0 wt.%, 2.0 to 8.0 wt.%, 2.0 to 7.0 wt.%, or 2.0 to 6.0 wt.%.

The nucleating agent may be, but is not necessarily limited to, P₂O₅ and/or a metal, like Cu. In one preferred embodiment, the nucleating agent is P₂O₅. The nucleating agent may be present in an amount in a range of 0.5 to 10 wt.%, like in a range 1.0 to 9.0 wt.%, 1.5 to 8.5 wt.% or 2.0 to 8.0 wt.%.

The glass or glass-ceramic may comprise one or more additional components. The one or more additional components may be one or more of:
0.0 to 5.0 wt.%, like in range of 0.0 to 4.0 wt.% or 0.0 to 3.5 wt.%, of Me¹(I)₂O,
0.0 to 12 wt.%, like in a range of 0.0 to 11 wt.% or 0.0 to 9.5 wt.%, of Me²(II)O,
0.0 to 5.0 wt.%, like in a range of 0.0 to 4.5 wt.% or 0.0 to 4.0 wt.%, of Me³(III)₂O₃,
0.0 to 8.0 wt.%, like in a range of 0.0 to 7.0 wt.% or 0.0 to 6.5 wt.%, of Me⁴(IV)O₂,
0.0 to 6.0 wt.%, like in a range of 0.0 to 5.5 wt.% or 0.0 to 5.0 wt.%, of Me⁵(V)₂O₅,
0.0 to 1.5 wt.%, like in a range of 0.0 to 1.0 wt.% or 0.0 to 0.5 wt.%, of Me⁶(VI)O₆,
0.0 to 1.0 wt.%, like in a range of 0.0 to 0.5 wt.% or 0.0 to 0.3 wt.%, mixed valence metal oxide,
0.0 to 1.5 wt.%, like in a range of 0.0 to 1.0 wt.% or 0.0 to 0.5 wt.%, of F,
wherein
Me¹(I)₂O is selected from one valent metal oxides (Me¹(I)₂O) and mixtures thereof, wherein Li₂O is excluded,
Me²(II)O is selected from two valent metal oxides and mixtures thereof,
Me³(III)₂O₃ is selected from three valent metal oxides and mixtures thereof, wherein Al₂O₃ is excluded,
Me⁴(IV)O₂ is selected from four valent metal oxides and mixtures thereof, wherein SiOz is excluded,
Me⁵(V)₂O₅ is selected from five valent metal oxides and mixtures thereof, and
Me⁶(VI)O₆ is selected from six valent metal oxides and mixtures thereof.

The one valent metal oxides (Me¹(I)₂O) may be selected from Na₂O, K₂O, Rb₂O, Cs₂O and mixtures thereof. The overall amount of one valent metal oxides (Me¹(I)₂O) in the glass or glass-ceramic, i.e., including Li₂O, may be equal to or less than 25 wt.% or 26 wt.%. The glass or glass-ceramic may comprise a weight amount of Na₂O and/or K₂O of less than 2.0 wt.%, less than 1.0 wt.%, or less than 0.5 wt.%. The glass or glass-ceramic may comprise a weight amount of one valent metal oxides (Me¹(I)₂O) other than Li₂O of less than 2.0 wt.%, less than 1.0 wt.%, or less than 0.5 wt.%.

The glass or glass-ceramic may essentially be free of (or may be free of) Na₂O, K₂O, Rb₂O, or Cs₂O. The glass or glass-ceramic may essentially be free of (or may be free of) one valent metal oxides (Me¹(I)₂O) other than Li₂O. In one embodiment, the glass or glass-ceramic is essentially free of (or completely free of) Na₂O. In one embodiment, the glass or glass-ceramic is essentially free of (or completely free of) Na₂O and K₂O. A particularly low amount or the absence of one or all of one valent metal oxides (Me¹(I)₂O) other than Li₂O can have a positive effect on chemical resistance and/or fracture resistance.

The two valent metal oxides (Me²(II)O) may be selected from, but are not limited to, CaO, BaO, MgO, SrO, ZnO, SnO and mixtures thereof. The glass or glass-ceramic may essentially be free of (or may be free of) CaO, BaO, MgO, SrO, ZnO, or SnO. The glass or glass-ceramic may essentially be free of (or may be free of) two valent metal oxides (Me²(II)O).

The three valent metal oxides (Me³(III)₂O₃) may be selected from, but are not limited to, B₂O₃, ErzOs, La₂O₃, Y₂O₃, Yb₂O₃, Ga₂O₃, In₂O₃, GdzOs, Eu₂O₃, DyzOs, and mixtures thereof. The overall amount of three valent metal oxides (Me³(III)₂O₃) in the glass or glass-ceramic, i.e., including Al₂O₃, may be equal to or less than 15 wt.% or 16 wt.%.

The four valent metal oxides (Me⁴(IV)O₂) may be selected from, but are not limited to, ZrO₂, TiO₂, SnO₂, GeO₂, and mixtures thereof.

The five valent metal oxides (Me⁵(V)₂O₅) may be selected from, but are not limited to, Ta₂O₅, Nb₂O₅, Nd₂O₅, and mixtures thereof. P₂O₅ is not a five valent metal oxide (Me⁵(V)₂O₅) in the meaning of the present disclosure.

The mixed valence metal oxide may be, but is not limited to, one or more mixed three/four valent metal oxides (Me^{3/4}(III,IV)₄O₇), like WOs, MoOs and Tb₄O₇.

In several embodiments, the glass or glass-ceramic comprises one or more additional components selected from:
0.1 to 5.0 wt.%, like in range of 0.3 to 4.0 wt.% or 0.5 to 3.5 wt.%, of Me¹(I)₂O,
0.1 to 12 wt.%, like in a range of 0.5 to 11 wt.% or 1.0 to 9.5 wt.%, of Me²(II)O,
0.1 to 5.0 wt.%, like in a range of 0.1 to 4.5 wt.% or 0.1 to 4.0 wt.%, of Me³(III)₂O₃,
0.1 to 8.0 wt.%, like in a range of 1.0 to 7.0 wt.% or 2.0 to 6.5 wt.%, of Me⁴(IV)O₂,
0.02 to 6.0 wt.%, like in a range of 0.1 to 5.5 wt.% or 1.0 to 5.0 wt.%, of Me⁵(V)₂O₅,
0.02 to 1.0 wt.%, like in a range of 0.05 to 0.5 wt.% or 0.1 to 0.3 wt.%, mixed valence metal oxide,
0.1 to 1.5 wt.%, like in a range of 0.2 to 1.0 wt.% or 0.2 to 0.5 wt.%, of F,
wherein
Me¹(I)₂O is selected from one valent metal oxides (Me¹(I)₂O) and mixtures thereof, wherein Li₂O is excluded,
Me²(II)O is selected from two valent metal oxides and mixtures thereof,
Me³(III)₂O₃ is selected from three valent metal oxides and mixtures thereof, wherein Al₂O₃ is excluded,
Me⁴(IV)O₂ is selected from four valent metal oxides and mixtures thereof, wherein SiOz is excluded,
Me⁵(V)₂O₅ is selected from five valent metal oxides and mixtures thereof, and
Me⁶(VI)O₆ is selected from six valent metal oxides and mixtures thereof.

In many embodiments, the glass or glass-ceramic additionally comprises 0.1 to 7.0 wt.%, like in a range of 0.1 to 6.5 wt.% or 0.5 to 6.0 wt.%, of Me⁴(IV)O₂.

For example, the glass or glass-ceramic may comprise one or more additional components selected from:
0.0 to 5.0 wt.%, like in a range of 0.0 to 4.0 wt.% or 0.0 to 3.0 wt.%, of Na₂O,
0.0 to 5.0 wt.%, like in a range of 0.0 to 3.5 wt.% or 0.0 to 2.5 wt.%, of K₂O,
0.0 to 6.0 wt.%, like in a range of 0.0 to 5.0 wt.% or 0.0 to 4.5 wt.%, of MgO,
0.0 to 7.0 wt.%, like in a range of 0.0 to 5.0 wt.% or 0.0 to 5.5 wt.%, of CaO,
0.0 to 11.0 wt.%, like in a range of 0.0 to 10.0 wt.% or 0.0 to 9.5 wt.%, of SrO,
0.0 to 9.0 wt.%, like in a range of 0.0 to 8.0 wt.% or 0.0 to 7.5 wt.%, of ZnO,
0.0 to 5.0 wt.%, like in a range of 0.0 to 4.0 wt.% or 0.0 to 3.5 wt.%, of La₂O₃,
0.0 to 5.0 wt.%, like in a range of 0.0 to 4.0 wt.% or 0.0 to 3.5 wt.%, of Y₂O₃,
0.0 to 0.8 wt.%, like in a range of 0.0 to 0.5 wt.% or 0.0 to 0.3 wt.%, of Er₂O₃,
0.0 to 4.0 wt.%, like in a range of 0.0 to 3.0 wt.% or 0.0 to 2.5 wt.%, of Gd₂O₃,
0.0 to 2.0 wt.%, like in a range of 0.0 to 1.5 wt.% or 0.0 to 1.2 wt.%, of Eu₂O₃,
0.0 to 4.0 wt.%, like in a range of 0.0 to 3.0 wt.% or 0.0 to 2.5 wt.%, of DyzOs,
0.0 to 8.0 wt.%, like in a range of 0.0 to 7.0 wt.% or 0.0 to 6.5 wt.%, of ZrO₂,
0.0 to 3.0 wt.%, like in a range of 0.0 to 2.0 wt.% or 0.0 to 1.5 wt.%, of SnO₂,
0.0 to 5.0 wt.%, like in a range of 0.0 to 4.0 wt.% or 0.0 to 3.5 wt.%, of CeO₂,
0.0 to 0.6 wt.%, like in a range of 0.0 to 0.4 wt.% or 0.0 to 0.2 wt.%, of V₂O₅,
0.0 to 7.0 wt.%, like in a range of 0.0 to 6.0 wt.% or 0.0 to 5.5 wt.%, of Ta₂O₅,
0.0 to 7.0 wt.%, like in a range of 0.0 to 6.0 wt.% or 0.0 to 5.5 wt.%, of Nb₂O₅,
0.0 to 0.6 wt.%, like in a range of 0.0 to 0.4 wt.% or 0.0 to 0.25 wt.%, of Tb₄O₇.

In many embodiments, the glass or glass-ceramic comprises one or more additional components selected from:
0.1 to 5.0 wt.%, like in a range of 0.3 to 4.0 wt.% or 0.5 to 3.0 wt.%, of Na₂O,
0.1 to 5.0 wt.%, like in a range of 0.3 to 3.5 wt.% or 0.3 to 2.5 wt.%, of K₂O,
0.1 to 6.0 wt.%, like in a range of 0.1 to 5.0 wt.% or 0.3 to 4.5 wt.%, of MgO,
0.1 to 7.0 wt.%, like in a range of 1.0 to 5.0 wt.% or 3.0 to 5.5 wt.%, of CaO,
0.1 to 11.0 wt.%, like in a range of 1.0 to 10.0 wt.% or 4.0 to 9.5 wt.%, of SrO,
0.1 to 9.0 wt.%, like in a range of 0.5 to 8.0 wt.% or 0.5 to 7.5 wt.%, of ZnO,
0.1 to 5.0 wt.%, like in a range of 0.5 to 4.0 wt.% or 1.0 to 3.5 wt.%, of LazOs,
0.1 to 5.0 wt.%, like in a range of 0.5 to 4.0 wt.% or 1.0 to 3.5 wt.%, of Y₂O₃,
0.1 to 0.8 wt.%, like in a range of 0.1 to 0.5 wt.% or 0.1 to 0.3 wt.%, of Er₂O₃,
0.1 to 4.0 wt.%, like in a range of 0.1 to 3.0 wt.% or 0.5 to 2.5 wt.%, of Gd₂O₃,
0.1 to 2.0 wt.%, like in a range of 0.1 to 1.5 wt.% or 0.1 to 1.2 wt.%, of Eu₂O₃,
0.1 to 4.0 wt.%, like in a range of 0.1 to 3.0 wt.% or 0.5 to 2.5 wt.%, of Dy₂O₃,
0.1 to 7.0 wt.%, like in a range of 0.1 to 6.5 wt.% or 0.1 to 6.0 wt.%, of ZrO₂,
0.1 to 3.0 wt.%, like in a range of 0.1 to 2.0 wt.% or 0.5 to 1.5 wt.%, of SnO₂,
0.1 to 5.0 wt.%, like in a range of 0.1 to 4.0 wt.% or 0.1 to 3.5 wt.%, of CeO₂,
0.1 to 0.6 wt.%, like in a range of 0.1 to 0.4 wt.% or 0.1 to 0.2 wt.%, of V₂O₅,
0.1 to 7.0 wt.%, like in a range of 0.5 to 6.0 wt.% or 1.0 to 5.5 wt.%, of Ta₂O₅,
0.1 to 7.0 wt.%, like in a range of 0.5 to 6.0 wt.% or 1.0 to 5.5 wt.%, of Nb₂O₅,
0.1 to 0.6 wt.%, like in a range of 0.1 to 0.4 wt.% or 0.1 to 0.25 wt.%, of Tb₄O₇.

In many embodiments, the glass or glass-ceramic additionally comprises 0.1 to 7.0 wt.%, like in a range of 0.1 to 6.5 wt.% or 0.5 to 6.0 wt.%, of ZrO₂.

In certain embodiment, the glass is a solid glass body or a solid glass-ceramic body comprising:
62 to 74 wt.%, like in a range of 64 to 72 wt.% or 66 to 70 wt.%, of SiO₂,
4.0 to 10 wt.% of Li₂O,
4.0 to 9.0 wt.%, like in a range of 5.0 to 9.0 wt.% or 6.0 to 8.0 wt.%, of Al₂O₃, and
a nucleating agent (typically P₂O₅).

In certain embodiment, the glass is a solid glass body or a solid glass-ceramic body comprising:
62 to 74 wt.%, like in a range of 64 to 72 wt.% or 66 to 70 wt.%, of SiO₂,
4.0 to 10 wt.% of Li₂O,
4.0 to 9.0 wt.%, like in a range of 5.0 to 9.0 wt.% or 6.0 to 8.0 wt.%, of Al₂O₃,
1.0 to 6.0 wt.%, like in a range of 2.0 to 5.0 wt.% or 3.0 to 4.0 wt.%, a nucleating agent (typically P₂O₅), and optionally
0.1 to 5.0 wt.%, like in range of 0.3 to 4.0 wt.% or 0.5 to 3.5 wt.%, of Me¹(I)₂O (e.g., Na₂O and K₂O),
0.1 to 5.0 wt.%, like in a range of 0.5 to 4.0 wt.% or 1.0 to 3.0 wt.%, of Me²(II)O (e.g., ZnO and MgO),
0.0 to 0.1 wt.%, like 0.1 wt.%, of Me³(III)₂O₃ (e.g., Er₂O₃),
0.1 to 6.0 wt.%, like in a range of 1.0 to 6.0 wt.% or 2.0 to 4.0 wt.%, of Me⁴(IV)O₂ (e.g., ZrO₂ and CeO₂),
0.1 to 0.5 wt.%, like in a range of 0.1 to 0.4 wt.% or 0.1 to 0.3 wt.%, of Me⁵(V)₂O₅ (e.g., V₂O₅), and
0.1 to 0.5 wt.%, like in a range of 0.1 to 0.4 wt.% or 0.1 to 0.3 wt.%, mixed valence metal oxide (e.g., Tb₄O₇).

The glass or the glass-ceramic may be colored. For example, when the glass is a solid glass body or a solid glass-ceramic body, the solid glass body or a solid glass-ceramic body may comprise coloring metal ions, like ions of Fe, Mn, Cr, Pr, Tb, Er, Yb, Ce, Co, Ni, Nd, Cu, Bi, Au, Ag and any mixture thereof. On the other hand, when the glass or glass-ceramic is a powder, it may be that the powder it essentially uncolored (although this is not necessary).

The composition of the glass or glass-ceramic may be adjusted such that the glass or glass-ceramic is suitable to form one or more crystal phases according to the embodiments of the lithium alumosilicate glass-ceramic of the present invention.

The composition of the glass or glass-ceramic may be adjusted such that the glass or glass-ceramic is suitable to form a keatite s.s. crystal phase as a main crystal phase in a lithium alumosilicate glass-ceramic obtained therefrom. In said case, the weight amount of Al₂O₃ is typically adjusted to a higher value. For example, the weight amount of Al₂O₃ may be selected from a weight amount in a range of 5.0 to 15 wt.% or 7.0 to 15 wt.%. In one embodiment, the composition of the glass or glass-ceramic is adjusted such that the glass or glass-ceramic is suitable to form a keatite s.s. crystal phase as a main crystal phase, wherein the composition comprises: 62 to 76 wt.%, like in a range of 64 to 74 wt.%, of SiO₂, 7.0 to 18 wt.%, like in a range of 8.5 to 16 wt.%, of Li₂O, 4.0 to 15 wt.%, like in a range of 5.0 to 15 wt.%, of Al₂O₃.

Alternatively, the composition of the glass or glass-ceramic may be adjusted such that the glass or glass-ceramic is suitable to form a keatite s.s. crystal phase as a minor crystal phase in a lithium alumosilicate glass-ceramic obtained therefrom. In said case, the weight amount of Al₂O₃ is often selected from a lower value. For example, the weight amount of Al₂O₃ may be selected from a weight amount in a range of 2.0 to 8.0 wt.%, 2.0 to 7.0 wt.%, or 2.0 to 6.0 wt.%. In certain embodiments, the composition of the glass or glass-ceramic is adjusted such that the glass or glass-ceramic is suitable to form a quartz s. s., Li₂Si₂O₅ or Li₂SiO₃ is a main crystal phase, and a keatite solid solution crystal phase is a minor crystal phase, in a lithium alumosilicate glass-ceramic obtained therefrom. In one embodiment, the composition of the glass or glass-ceramic is adjusted such that the glass or glass-ceramic is suitable to form Li₂Si₂O₅ as the main crystal phase and the keatite s.s. crystal phase as a minor crystal phase, and wherein the composition comprises: 64 to 78 wt.%, like in a range of 66 to 76 wt.%, of SiO₂, 11 to 20 wt.%, like in a range of 13 to 18 wt.%, of Li₂O, 2.0 to 9.0 wt.%, like in a range of 2.5 to 7.5 wt.%, of Al₂O₃. In one embodiment, the composition of the glass or glass-ceramic is adjusted such that the glass or glass-ceramic is suitable to form a quartz s.s. crystal phase as the main crystal phase and the keatite s.s. crystal phase as a minor crystal phase, and wherein the composition comprises: 60 to 78 wt.%, like in a range of 62 to 78 wt.%, of SiO₂, 8.5 to 17 wt.%, like in a range of 8.5 to 15 wt.%, of Li₂O, 2.0 to 13 wt.%, like in a range of 3.5 to 11 wt.%, of Al₂O₃.

### 2. Structure and shape

The glass or glass-ceramic may be a glass. In an alternative, the glass or glass-ceramic may be a glass-ceramic. The glass-ceramic may comprise one or more crystal phases which is/are not a keatite s.s. crystal phase such as, but not limited to, lithium metasilicate. For example, the glass-ceramic may comprise a crystal phase which may be converted to a keatite s.s. crystal phase.

The glass or glass ceramic may have a glass transition temperature Tg in a range of 430 to 550°C, like in a range of 440 to 540°C. The glass transition temperature Tg may be determined according to ISO 11357-2.

The glass or glass-ceramic may be a glass which is a solid glass body or a solid glass-ceramic body.

The solid glass body may be obtained by freezing a molten glass, e.g., in a mold. The solid glass body may have the shape of a dental blank, like a dental mill blank or a dental press blank. The solid glass body may also have the shape of a part of a dental blank, like a layer of a dental blank.

The solid glass-ceramic body may be obtained by (partially) crystallizing a solid glass body, like a solid glass body as described herein. The (partial) crystallizing may be carried out at a (maximum) temperature below a temperature range for crystallizing a keatite s.s. crystal phase. The (partial) crystallizing may be carried out at a temperature for crystallizing lithium metasilicate. For example, the (partial) crystallizing may be carried out at a temperature in a range of 650 to 750°C, like about 700°C. The solid glass-ceramic body may have the shape of a dental blank, like a dental mill blank or a dental press blank. The solid glass-ceramic body may also have the shape of a part of a dental blank, like a layer of a dental blank.

In an alternative, the glass or glass-ceramic is a powder, like a glass powder, a glass-ceramic powder, or a mixture of both. The volume-based median particle size d₅₀ of the glass powder and/or glass-ceramic powder may be in a range of 0.1 to 100 µm, like in a range of 1 to 50 µm. The volume-based top cut particle size d₉₈ of the glass powder and/or glass-ceramic powder may be below 90 µm, like below 63 µm. The volume-based median particle size d₅₀ and the volume-based top cut particle size d₉₈ may be determined by laser diffraction, e.g., according to ISO 13320 (ISO 13320:2009).

The glass powder and/or glass-ceramic powder may be present in dry form, like in form of a dry blend additionally comprising one or more additives. The dry blend may be a particulate mixture or may be in the form of granules, and preferably is in the form of granules. The dry blend (e.g., the granules) may comprise the glass powder and/or glass-ceramic powder in an amount of at least 90 wt.%, based on the total weight of the dry blend. The one or more additives may be present in the dry blend (e.g., granules) in an amount in a range of 0.1 to 10.0 wt.%, like 0.3 to 5.0 wt.%, based on the total weight of the dry blend. The one or more additives typically comprise one or more binders (e.g., polyvinyl alcohols and cellulose derivatives, such as sodium carboxymethylcellulose). The one or more binders may be present in the dry blend in an amount in a range of 0.1 to 5.0 wt.%, like 0.3 to 3.0 wt.%, based on the total weight of the dry blend. Further additives may be, but are not limited to, one or more fluorescent or non-fluorescent pigments (e.g., doped spinels, doped zirconium oxides, zirconium oxide, doped zirconium silicates, doped yttrium silicates or tin oxide) or pressing aids (e.g., polyethylene glycols or stearates). The one or more pressing aids may be present in the dry blend in an amount in a range of 0.1 to 3.0 wt.%, like 0.1 to 1.0 wt.%, based on the total weight of the dry blend.

Alternatively, the glass powder and/or glass-ceramic powder may be present in liquid form, and particularly in the form of a suspension. The further ingredients of the suspension may depend on the use of the suspension.

For example, the suspension may be for use in die casting or slip casting. In such case, the suspension may be an aqueous suspension. The suspension may comprise the glass powder and/or glass ceramic powder in an amount in a range of 30 to 90 wt.%, like 40 to 70%, based on the total weight of the suspension. The suspension may comprise one or more additives (e.g., one or more fluorescent or non-fluorescent pigments, one or more pressing aids and/or one or more binders) in an amount in a range of 0.1 to 10.0 wt.%, like 0.3 to 5.0 wt.%, based on the total dry weight of the suspension. Suitable pigments, pressing aids and binders are known to the skilled person, and may be, but are not limited, to those described above. The one or more binders may be present in an amount in a range of 0.1 to 5.0 wt.%, like 0.3 to 3.0 wt.%, based on the total weight of the dry weight of the suspension. The one or more pressing aids may be present in an amount in a range of 0.1 to 3.0 wt.%, like 0.1 to 1.0 wt.%, based on the total dry weight of the suspension. Additionally or alternatively, the suspension may comprise one or more auxiliary agents, like one or more rheology modifiers (e.g., xanthans or starches), one or more dispersants (e.g., polymers or lecithins), one or more buffers and/or pH adjusting agents (e.g., acids, like acetic acid or hydrochloric acid), and the like. The suspension may comprise the one or more auxiliary agents in an amount in a range of 0.1 to 3.0 wt.%, based on the total weight of the suspension.

Alternatively, the suspension may be for use in an additive manufacturing process such as stereo lithography, inkjet printing, screen printing, powder bed printing or fused deposition modeling (fused filament fabrication). In one embodiment, the glass powder and/or glass-ceramic powder is provided in form of a suspension which is suitable for use in an additive manufacturing process, and preferably a stereolithography process. In said embodiment, the suspension typically comprises the glass powder and/or glass-ceramic powder, an organic monomer solution, and a photo initiator.

When present in form of a powder, the glass or glass-ceramic may be obtained by a process as described herein, e.g., in section II.4.1.1.

In one aspect, the present invention provides a use of a glass or glass-ceramic according to the present invention for preparing a lithium alumosilicate glass-ceramic comprising a keatite s.s. crystal phase, preferably a lithium alumosilicate glass-ceramic according to the present invention. The glass or glass-ceramic according to the present invention is thus useful for preparing a lithium alumosilicate glass-ceramic according to the present invention. The lithium alumosilicate glass-ceramic is described in more detail herein below.

### II. The lithium alumosilicate glass-ceramic for a dental body

In one aspect, the present invention provides a lithium alumosilicate glass-ceramic for a dental body, wherein the lithium aluminosilicate glass-ceramic comprises
60 to 78 wt.% of SiO₂,
8.5 to 24 wt.% of Li₂O,
2.0 to 15 wt.% of Al₂O₃, and a nucleating agent, and wherein the lithium alumosilicate glass-ceramic comprises a keatite solid solution crystal phase.

The lithium alumosilicate glass-ceramic is for a dental body. This encompasses that the lithium alumosilicate glass-ceramic is suitable for use as a glass-ceramic body for a dental body, or as a part of such a glass-ceramic body. The lithium alumosilicate glass-ceramic may be obtained from a glass or glass-ceramic according to the present invention.

### 1. Crystal phase(s) and physical properties

The lithium alumosilicate glass-ceramic comprises a keatite s.s. crystal phase. The lithium alumosilicate may comprise the keatite s.s. crystal phase in an amount in a range of 2.5 to 50 wt.%, like in a range of 5.0 to 50 wt.% or 7.5 to 48 wt.% or 10 to 45 wt.%. In certain embodiments, the lithium alumosilicate glass-ceramic comprises the keatite s.s. crystal phase in an amount in a range of 2.5 to 25 wt.%, like in a range of 5.0 to 25 wt.% or 7.5 to 22 wt.% or 10 to 20 wt.%. In certain embodiments, the lithium alumosilicate glass-ceramic comprises the keatite s.s. crystal phase in an amount in a range of 20 to 50 wt.%, like in a range of 22 to 50 wt.% or 25 to 48 wt.% or 25 to 45 wt.%. In one embodiment, the lithium alumosilicate glass-ceramic comprises the keatite s.s. crystal phase in an amount in a range of 35 to 50 wt.%, like in a range of 40 to 48 wt.%. In an alternative embodiment, the lithium alumosilicate glass-ceramic comprises the keatite s.s. crystal phase in an amount in a range of 22 to 30 wt.%, like in a range of 22 to 28 wt.%. In another alternative embodiment, the lithium alumosilicate glass-ceramic comprises the keatite s.s. crystal phase in an amount in a range of 10 to 20 wt.%, like in a range of 12 to 20 wt.%. All weight percentages of crystal phases described in this section are based on the total weight of the lithium alumosilicate glass-ceramic, unless explicitly stated otherwise. The weight amount of the keatite s.s. crystal phase (and other crystal phases described herein) may be determined using the Rietveld method as described herein in the measuring methods.

In addition to the keatite s.s. crystal phase, the lithium alumosilicate glass-ceramic typically comprises one or more other crystal phases. Other crystal phases may be, but are not limited to, Li₃PO₄, Li₂Si₂O₅, quartz s.s., Li₂SiO₃, petalite, LiSr(PO₄), Li₂ZnSiO₄, LiZnPO₄, Li₄Zn(PO₄)₂, CeO₂, and Ca₅(PO₄)₃F.

The keatite s.s. crystal phase may be the main crystal phase of the lithium alumosilicate glass-ceramic. In certain embodiments, the keatite s.s. crystal phase is the main crystal phase of the lithium alumosilicate glass-ceramic, and the lithium alumosilicate glass-ceramic comprises the keatite s.s. crystal phase in an amount in a range of 20 to 50 wt.%, like in a range of 22 to 50 wt.% or 25 to 48 wt.% or 25 to 45 wt.%.

When the keatite s.s. crystal phase is the main crystal phase, the lithium alumosilicate glass-ceramic typically comprises one or more minor crystal phases. Minor crystal phases may be, but are not limited to, Li₃PO₄, Li₂Si₂O₅, quartz s.s., Li₂SiO₃, petalite, LiSr(PO₄), Li₂ZnSiO₄, LiZnPO₄, Li₄Zn(PO₄)₂, CeO₂, and Ca₅(PO₄)₃F. In many embodiments, when the keatite s.s. crystal phase is the main crystal phase, the lithium alumosilicate glass-ceramic comprises one or more of Li₃PO₄, Li₂Si₂O₅, quartz s.s., and Li₂SiO₃, as one or more minor crystal phases, optionally in combination with one or more additional minor crystal phases (e.g., selected from petalite, LiSr(PO₄), Li₂ZnSiO₄, LiZnPO₄, Li₄Zn(PO₄)₂, CeO₂, and Ca₅(PO₄)₃F). In certain embodiments, when the keatite s.s. crystal phase is the main crystal phase, the lithium alumosilicate glass-ceramic comprises Li₃PO₄, optionally in combination with Li₂Si₂O₅, as minor crystal phase(s), further optionally in combination with one or more additional minor crystal phases (e.g., selected from quartz s.s., Li₂SiO₃, petalite, LiSr(PO₄), Li₂ZnSiO₄, LiZnPO₄, Li₄Zn(PO₄)₂, CeO₂, and Ca₅(PO₄)₃F). In certain embodiments, when the keatite s.s. crystal phase is the main crystal phase, the lithium alumosilicate glass-ceramic comprises Li₃PO₄, Li₂Si₂O₅, and quartz s.s. as minor crystal phases, optionally in combination with one or more additional minor crystal phases (e.g., selected from Li₂SiO₃, petalite, LiSr(PO₄), Li₂ZnSiO₄, LiZnPO₄, Li₄Zn(PO₄)₂, CeO₂, and Ca₅(PO₄)₃F). In certain embodiments, when the keatite s.s. crystal phase is the main crystal phase, the lithium alumosilicate glass-ceramic comprises Li₃PO₄, and Li₂SiO₃ as minor crystal phases, optionally in combination with one or more additional minor crystal phases (e.g., selected from Li₂Si₂O₅, quartz s.s., petalite, LiSr(PO₄), Li₂ZnSiO₄, LiZnPO₄, Li₄Zn(PO₄)₂, CeO₂, and Ca₅(PO₄)₃F).

In selected embodiments, when the keatite s.s. crystal phase is the main crystal phase, the lithium alumosilicate glass-ceramic comprises minor crystal phases according to one of the embodiments 1 a to 1 k as defined in table 1 herein below.

**Table 1**

| **Embodiment** | **Minor crystal phases** |
|---|---|
| 1a | Li₂Si₂O₅; Li₃PO₄ |
| 1b | petalite, quartz s.s., Li₂Si₂O₅, Li₂SiO₃, Li₃PO₄ |
| 1c | quartz s.s.; Li₂Si₂O₅; Li₃PO₄ |
| 1d | Li₃PO₄ |
| 1e | Li₃PO₄; Li₂SiO₃ |
| 1f | quartz s.s.; Li₂Si₂O₅; Li₂SiO₃; Li₃PO₄ |
| 1g | Li₂SiO₃; quartz s.s.; Li₃PO₄ |
| 1h | Li₂Si₂O₅; Li₂ZnSiO₄; LiZnPO₄; Li₃PO₄ |
| 1i | Li₂Si₂O₅; Li₃PO₄; Li₂Zn(SiO₄); Li₄Zn(PO₄)₂ |
| 1j | Li₂Si₂O₅; Li₃PO₄; Li₂Zn(SiO₄) |
| 1k | Li₂Si₂O₅; CeO₂; Li₃PO₄; Li₂Zn(SiO₄) |

Alternatively, the keatite s.s. crystal phase is a minor crystal phase of the lithium alumosilicate glass-ceramic. In certain embodiments, the keatite s.s. crystal phase is a minor crystal phase of the lithium alumosilicate glass-ceramic, and the lithium alumosilicate glass-ceramic comprises the keatite s.s. crystal phase in an amount in a range of 2.5 to 25 wt.%, like in a range of 5.0 to 25 wt.% or 7.5 to 22 wt.% or 10 to 20 wt.%.

When the keatite s.s. crystal phase is a minor crystal phase, the lithium alumosilicate glass-ceramic may comprise a quartz s.s., Li₂Si₂O₅ or Li₂SiO₃ as the main crystal phase, and preferably either a quartz s.s. or Li₂Si₂O₅. In many embodiments, the keatite s.s. crystal phase is a minor crystal phase, and the lithium alumosilicate glass-ceramic comprises Li₂Si₂O₅ as the main crystal phase.

In certain embodiments, the lithium alumosilicate glass-ceramic comprises Li₂Si₂O₅ as the main crystal phase, and the keatite s.s. crystal phase and Li₃PO₄ as minor crystal phases, optionally in combination with a quartz s.s. as an additional minor crystal phase, and optionally in combination with one or more further additional minor crystal phases.

In certain embodiments, the lithium alumosilicate glass-ceramic comprises a quartz s.s. as the main crystal phase, and the keatite s.s. crystal phase and Li₃PO₄ as minor crystal phases, typically in combination with Li₂Si₂O₅ as an additional minor crystal phase, and optionally in combination with one or more further additional minor crystal phases.

In certain embodiments, the lithium alumosilicate glass-ceramic comprises Li₂SiO₃ as the main crystal phase, and the keatite s.s. crystal phase and Li₃PO₄ as minor crystal phases, and optionally in combination with one or more additional minor crystal phases.

In selected embodiments, the lithium alumosilicate glass-ceramic comprises the crystal phases according to one of the embodiments 2a to 2j as defined in table 2 herein below.

**Table 2**

| **Embodiment** | **Main crystal phase** | **Minor crystal phases** |
|---|---|---|
| 2a | Li₂Si₂O₅ | Keatite s.s.; quartz s.s.; Li₃PO₄ |
| 2b | Li₂Si₂O₅ | Keatite s.s.; quartz s.s.; Li₃PO₄; LiSr(PO₄) |
| 2c | Li₂Si₂O₅ | Keatite s.s.; quartz s.s.; Li₃PO₄; Li₂ZnSiO4 |
| 2d | Li₂Si₂O₅ | Keatite s.s.; Li₃PO₄ |
| 2e | Li₂Si₂O₅ | Keatite s.s.; Li₃PO₄; Li₂SiO₃ |
| 2f | Li₂Si₂O₅ | Keatite s.s.; Li₃PO₄; Ca₅(PO₄)₃F |
| 2g | Quartz s.s. | Li₂Si₂O₅; keatite s.s.; Li₃PO₄ |
| 2h | Quartz s.s. | Keatite s.s.; Li₂SiO₃; Li₃PO₄; Li₂ZnSiO₄ |
| 2i | Quartz s.s. | Li₂SiO₃; keatite s.s.; Li₂Si₂O₅; Li₃PO₄ |
| 2j | Li₂SiO₃ | Keatite s.s.; Li₃PO₄; |

The lithium alumosilicate glass-ceramic may comprise the crystal phases in specific weight amounts.

In one embodiment, the lithium alumosilicate glass-ceramic comprises the keatite s.s. crystal phase in an amount of 35 to 50 wt.%, like in a range of 40 to 50 wt.%, a Li₃PO₄ crystal phase in an amount of 2.0 to 10 wt.%, like in a range of 3.0 to 7.5 wt.%, and a Li₂SiO₃ crystal phase in an amount of 0.5 to 5.0 wt.%, like in a range of 1.0 to 3.0 wt.%.

In an alternative embodiment, the lithium alumosilicate glass-ceramic comprises the keatite s.s. crystal phase in an amount of 22 to 30 wt.%, like in a range of 22 to 28 wt.%, a Li₂Si₂O₅ crystal phase in an amount of 22.0 to 30 wt.%, like in a range of 22 to 28 wt.%, and a Li₃PO₄ crystal phase in an amount of 5.0 to 15 wt.%, like in a range of 8.0 to 13 wt.%.

In another alternative embodiment, the lithium alumosilicate glass-ceramic comprises a Li₂SiO₃ crystal phase in an amount of 42 to 55 wt.%, like in a range of 45 to 52 wt.%, the keatite s.s. crystal phase in an amount of 12.0 to 22 wt.%, like in a range of 14 to 20 wt.%, and a Li₃PO₄ crystal phase in an amount of 5.0 to 15 wt.%, like in a range of 8.0 to 12 wt.%.

In another alternative embodiment, the lithium alumosilicate glass-ceramic comprises a Li₂Si₂O₅ crystal phase in an amount of 42 to 55 wt.%, like in a range of 45 to 52 wt.%, the keatite s.s. crystal phase in an amount of 10 to 20 wt.%, like in a range of 12 to 18 wt.%, a Li₃PO₄ crystal phase in an amount of 3.0 to 12 wt.%, like in a range of 5.0 to 10 wt.%, and a quartz s.s. crystal phase in an amount of 1.0 to 10 wt.%, like in a range of 2.5 to 7.5 wt.%.

The lithium alumosilicate glass-ceramic may be characterized by specific mechanical, optical, and/or thermal properties.

The lithium alumosilicate glass-ceramic may have a specific fracture toughness (K_{1C}). The fracture toughness (K_{1C}) may be determined according to the Single Edge V-Notched Beam (SEVNB) method according to DIN EN ISO 6872, and particularly DIN EN ISO 6872:2015. The lithium alumosilicate glass-ceramic may have a fracture toughness (K_{1C}) of at least 1.5 MPa*m^{1/2}, at least 1.8 MPa*m^{1/2}, at least 2.0 MPa*m^{1/2}, at least 2.2 MPa*m^{1/2}, or even at least 2.4 MPa*m^{1/2}. The lithium alumosilicate glass-ceramic may have a fracture toughness (K_{1C}) of at most 3.2 MPa*m^{1/2}, at most 3.0 MPa*m^{1/2}, or at most 2.8 MPa*m^{1/2}. The lithium alumosilicate glass-ceramic may have a fracture toughness (K_{1C}) in a range of 1.5 to 3.2 MPa*m^{1/2}, 1.8 to 3.0 MPa*m^{1/2}, 2.0 to 3.0 MPa*m^{1/2}, or 2.0 to 2.8 MPa*m^{1/2} (e.g., in a range of 2.2 to 3.0 MPa*m^{1/2} or even in a range of 2.4 to 2.8 MPa*m^{1/2}).

In one embodiment, the lithium alumosilicate glass-ceramic comprises Li₂Si₂O₅ as a main crystal phase and the keatite s.s. crystal phase as a minor crystal phase, optionally in combination with Li₃PO₄ and/or quartz s.s. as other minor crystal phases, and the lithium alumosilicate glass-ceramic has a fracture toughness (K_{1C}) in a range of 1.5 to 3.2 MPa*m^{1/2}, 1.8 to 3.0 MPa*m^{1/2}, or 2.0 to 2.8 MPa*m^{1/2}, (e.g., in a range of 2.2 to 3.0 MPa*m^{1/2} or even in a range of 2.4 to 2.8 MPa*m^{1/2}).

The lithium alumosilicate glass-ceramic may have a specific coefficient of the thermal expansion (CTE). The coefficient of the thermal expansion may be determined using a dilatometer according to DIN EN ISO 6872, and particularly DIN EN ISO 6872:2015. The CTE as defined herein refers to the CTE determined in a temperature range of 100 to 500°C. The lithium alumosilicate glass-ceramic may have a CTE in a range of 3.5 to 9.5 ppm/K, like in a range of 4.0 to 9.2 ppm/K. In one embodiment, the lithium alumosilicate glass-ceramic has a CTE in a range of 3.5 to 6.5 ppm/K, like in a range of 4.0 to 5.0 ppm/K. In an alternative embodiment, the lithium alumosilicate glass-ceramic has a CTE in a range of 6.8 to 9.5 ppm/K, like in a range of 7.2 to 9.2 ppm/K.

The inventors found that the CTE of the lithium alumosilicate glass-ceramic can be adjusted by adjusting the weight amount of the keatite s.s. crystal phase. When the weight amount of the keatite s.s. crystal phase is increased, the CTE typically decreases and *vice versa.* When the lithium alumosilicate glass-ceramic comprises the keatite s.s. crystal phase as a main crystal phase, the CTE is typically comparatively low. On the other hand, when the lithium alumosilicate glass-ceramic comprises the keatite s.s. crystal phase as a minor crystal phase, the CTE is typically comparatively high.

In one embodiment, the lithium alumosilicate glass-ceramic comprises the keatite s.s. crystal phase as the main crystal phase, and the lithium alumosilicate glass-ceramic has a CTE in a range of 3.5 to 6.5 ppm/K, like in a range of 4.0 to 5.0 ppm/K. In one embodiment, the lithium alumosilicate glass-ceramic comprises the keatite s.s. crystal phase in an amount in a range of 38 to 50 wt.%, like in a range of 40 to 48 wt.%, and the lithium alumosilicate glass-ceramic has a CTE in a range of 3.5 to 6.5 ppm/K, like in a range of 4.0 to 5.0 ppm/K.

In one embodiment, the lithium alumosilicate glass-ceramic comprises Li₂Si₂O₅ as the main crystal phase, and the keatite s.s. crystal phase as a minor crystal phase, and the lithium alumosilicate glass-ceramic has a CTE in a range of 6.8 to 9.5 ppm/K, like in a range of 7.2 to 9.2 ppm/K.

The lithium alumosilicate glass-ceramic may have specific optical properties, like color and/or contrast ratio (opacity). The lithium alumosilicate glass-ceramic may have a specific contrast ratio (contrast ratio and opacity are used synonymously herein). The contrast ratio may be determined according to BS 5612, and particularly BS 5612:1978. The contrast ratio as described herein is determined using a test body having a thickness of 2.0 mm. The contrast ratio relates to the ratio of illuminance (Y) of a material when placed on a black background (Yb) to the illuminance of the same material when placed over a white background (Yw) (CR = Yb/Yw). The contrast ratio can be used to characterize the translucency of a material, i.e. the light transmission of a material expressed as the ratio of transmitted to incident light intensity. A contrast ratio of close to 0% may indicate that a given material is almost fully transparent, while a contrast ratio of 100% may indicate that a material is fully opaque.

The lithium alumosilicate glass-ceramic may have a contrast ratio in a range of 50 to 99.8%, like in a range of 55 to 99.8%. In one embodiment, the lithium alumosilicate glass-ceramic has a contrast ratio in a range of 50 to 75%, like in a range of 55 to 70%. In one embodiment, the lithium alumosilicate glass-ceramic has a contrast ratio in a range of 68 to 99.8%, like in a range of 75 to 99.8%.

The inventors found that the contrast ratio of the lithium alumosilicate glass-ceramic can be adjusted by adjusting the weight amount of the keatite s.s. crystal phase. When the weight amount of the keatite s.s. crystal phase is increased, the contrast ratio typically decreases and *vice versa.* Accordingly, a higher weight amount of the keatite s.s. crystal phase may provide the lithium alumosilicate glass-ceramic with a higher translucency or transparency. When the lithium alumosilicate glass-ceramic comprises the keatite s.s. crystal phase as a main crystal phase, the contrast ratio is typically comparatively low.

In one embodiment, the lithium alumosilicate glass-ceramic comprises the keatite s.s. crystal phase as the main crystal phase, and the lithium alumosilicate glass-ceramic has a contrast ratio in a range of 50 to 75%, like in a range of 55 to 70%. In one embodiment, the lithium alumosilicate glass-ceramic comprises the keatite s.s. crystal phase in an amount in a range of 38 to 50 wt.%, like in a range of 40 to 48 wt.%, and the lithium alumosilicate glass-ceramic and the lithium alumosilicate glass-ceramic has a contrast ratio in a range of 50 to 75%, like in a range of 60 to 70%.

In one embodiment, the lithium alumosilicate glass-ceramic comprises Li₂Si₂O₅ as the main crystal phase, and the keatite s.s. crystal phase as a minor crystal phase, and the lithium alumosilicate glass-ceramic has a contrast ratio in a range of 68 to 99.8%, like in a range of 75 to 99.8%.

### 2. Chemical composition

The lithium alumosilicate glass-ceramic comprises
60 to 78 wt.% of SiO₂,
8.5 to 24 wt.% of Li₂O,
2.0 to 15 wt.% of Al₂O₃, and
a nucleating agent.

All weight amounts described in the present section are referenced to the total weight of the lithium alumosilicate glass-ceramic, unless explicitly stated otherwise. The sum of the weight amounts of the components is selected such that a total amount of 100 wt.% is not exceeded.

The lithium alumosilicate glass-ceramic comprises 60 to 78 wt.% of SiO₂, like an amount in a range of 62 to 78 wt.%. For example, the glass or glass ceramic may comprise 62 to 69 wt.% of SiOz. The lithium alumosilicate glass-ceramic may comprise 70 to 78 wt.% of SiOz.

The lithium alumosilicate glass-ceramic comprises 8.5 to 24 wt.% of Li₂O. For example, the lithium alumosilicate glass-ceramic may comprise 8.5 to 22 wt.% of Li₂O. The lithium alumosilicate glass-ceramic may comprise 10 to 20 wt.% of Li₂O.

The lithium alumosilicate glass-ceramic comprises 2.0 to 15 wt.% of Al₂O₃. In many embodiments, the lithium alumosilicate glass-ceramic comprises 2.0 to 10.0 wt.%, like in a range of 2.0 to 9.0 wt.%, 2.0 to 8.0 wt.%, 2.0 to 7.0 wt.%, or 2.0 to 6.0 wt.%, of Al₂O₃. It is however also possible that the lithium alumosilicate glass-ceramic comprises 10 to 15 wt.% of Al₂O₃.

The molar ratio of SiO₂ to Li₂O may be in a range of 1.50 to 4.22, preferably in a range of 1.80 to 4.00, and more preferably in a range of 2.00 to 3.80.

The ratio of the weight amount of Li₂O to the weight amount of Al₂O₃ may be in a range of 0.5 to 5.7. In one embodiment, the ratio of the weight amount of Li₂O to the weight amount of Al₂O₃ is in a range of 0.5 to 0.9. In one embodiment, the ratio of the weight amount of Li₂O to the weight amount of Al₂O₃ is in a range of 1.0 to 1.5. In one embodiment, the ratio of the weight amount of Li₂O to the weight amount of Al₂O₃ is in a range of 1.6 to 5.7.

In one embodiment, the weight ratio of Li₂O to Al₂O₃ is in a range of 1.6 to 5.7, and the lithium alumosilicate glass-ceramic comprises a weight amount of one valent metal oxides other than Li₂O of less than 2.0 wt.%, less than 1.0 wt.%, or less than 0.5 wt.%. In one embodiment, a weight ratio of Li₂O to Al₂O₃ is in a range of 1.6 to 5.7, and the lithium alumosilicate glass-ceramic is essentially free of (or is free of) one valent metal oxides other than Li₂O. A ratio of the weight amount of Li₂O to Al₂O₃ in a range of 1.6 to 5.7, optionally in combination with a particularly low amount (or absence) of other one valent metal oxides, can provide desirable mechanical properties, like a particularly good fracture toughness, for the lithium alumosilicate glass-ceramic.

In one embodiment, the weight ratio of the Li₂O to Al₂O₃ is in a range of 1.6 to 5.7, and the weight amount of Al₂O₃ is in a range of 2.0 to 10.0 wt.%, like in a range of 2.0 to 9.0 wt.%, 2.0 to 8.0 wt.%, 2.0 to 7.0 wt.%, or 2.0 to 6.0 wt.%.

The nucleating agent may be, but is not necessarily limited to, P₂O₅ and/or a metal, like Cu. In one preferred embodiment, the nucleating agent is P₂O₅. The nucleating agent may be present in an amount in a range of 0.5 to 10 wt.%, like in a range 1.0 to 9.0 wt.%, 1.5 to 8.5 wt.% or 2.0 to 8.0 wt.%.

The lithium alumosilicate glass-ceramic may comprise one or more additional components. The one or more additional components may be one or more of:
0.0 to 5.0 wt.%, like in range of 0.0 to 4.0 wt.% or 0.0 to 3.5 wt.%, of Me¹(I)₂O,
0.0 to 12 wt.%, like in a range of 0.0 to 11 wt.% or 0.0 to 9.5 wt.%, of Me²(II)O,
0.0 to 5.0 wt.%, like in a range of 0.0 to 4.5 wt.% or 0.0 to 4.0 wt.%, of Me³(III)₂O₃,
0.0 to 8.0 wt.%, like in a range of 0.0 to 7.0 wt.% or 0.0 to 6.5 wt.%, of Me⁴(IV)O₂,
0.0 to 6.0 wt.%, like in a range of 0.0 to 5.5 wt.% or 0.0 to 5.0 wt.%, of Me⁵(V)₂O₅,
0.0 to 1.5 wt.%, like in a range of 0.0 to 1.0 wt.% or 0.0 to 0.5 wt.%, of Me⁶(VI)O₆,
0.0 to 1.0 wt.%, like in a range of 0.0 to 0.5 wt.% or 0.0 to 0.3 wt.%, mixed valence metal oxide,
0.0 to 1.5 wt.%, like in a range of 0.0 to 1.0 wt.% or 0.0 to 0.5 wt.%, of F,
wherein
Me¹(I)₂O is selected from one valent metal oxides (Me¹(I)₂O) and mixtures thereof, wherein Li₂O is excluded,
Me²(II)O is selected from two valent metal oxides and mixtures thereof,
Me³(III)₂O₃ is selected from three valent metal oxides and mixtures thereof, wherein Al₂O₃ is excluded,
Me⁴(IV)O₂ is selected from four valent metal oxides and mixtures thereof, wherein SiOz is excluded,
Me⁵(V)₂O₅ is selected from five valent metal oxides and mixtures thereof, and
Me⁶(VI)O₆ is selected from six valent metal oxides and mixtures thereof.

The one valent metal oxides (Me¹(I)₂O) may be selected from Na₂O, K₂O, Rb₂O, Cs₂O and mixtures thereof. The overall amount of one valent metal oxides (Me¹(I)₂O) in the lithium alumosilicate glass-ceramic, i.e., including Li₂O, may be equal to or less than 25 wt.% or 26 wt.%. The lithium alumosilicate glass-ceramic may essentially be free of (or may be free of) Na₂O, K₂O, Rb₂O, or CszO. The lithium alumosilicate glass-ceramic may comprise a weight amount of Na₂O and/or K₂O of less than 2.0 wt.%, less than 1.0 wt.%, or less than 0.5 wt.%. The lithium alumosilicate glass-ceramic may comprise a weight amount of one valent metal oxides (Me¹(I)₂O) other than Li₂O of less than 2.0 wt.%, less than 1.0 wt.%, or less than 0.5 wt.%. The lithium alumosilicate glass-ceramic may essentially be free of (or may be free of) one valent metal oxides (Me¹(I)₂O) other than Li₂O. In one specific embodiment, the lithium alumosilicate glass-ceramic is essentially free of (or completely free of) Na₂O. In one specific embodiment, the lithium alumosilicate glass-ceramic is essentially free of (or completely free of) Na₂O and K₂O. A particularly low amount or the absence of one or all of one valent metal oxides (Me¹(I)₂O) other than Li₂O can have a positive effect on chemical resistance and/or fracture toughness.

The two valent metal oxides (Me²(II)O) may be selected from, but are not limited to, CaO, BaO, MgO, SrO, ZnO, SnO and mixtures thereof. The lithium alumosilicate glass-ceramic may essentially be free of (or may be free of) CaO, BaO, MgO, SrO, ZnO, or SnO. The lithium alumosilicate glass-ceramic may essentially be free of (or may be free of) two valent metal oxides (Me²(II)O).

The three valent metal oxides (Me³(III)₂O₃) may be selected from, but are not limited to, B₂O₃, ErzOs, La₂O₃, Y₂O₃, Yb₂O₃, Ga₂O₃, In₂O₃, GdzOs, Eu₂O₃, DyzOs, and mixtures thereof. The overall amount of three valent metal oxides (Me³(III)₂O₃) in the lithium alumosilicate glass-ceramic, i.e., including Al₂O₃, may be equal to or less than 15 wt.% or 16 wt.%.

The four valent metal oxides (Me⁴(IV) O₂) may be selected from, but are not limited to, ZrO₂, TiO₂, SnO₂, GeO₂, and mixtures thereof.

The five valent metal oxides (Me⁵(V)₂O₅) may be selected from, but are not limited to, Ta₂O₅, Nb₂O₅, Nd₂O₅, and mixtures thereof. P₂O₅ is not a five valent metal oxide (Me⁵(V)₂O₅) in the meaning of the present disclosure.

The mixed valence metal oxide may be, but is not limited to, one or more mixed three/four valent metal oxides (Me^{3/4}(III, IV)₄O₇), like WOs, MoO₃ and Tb₄O₇.

In several embodiments, the lithium alumosilicate glass-ceramic comprises one or more additional components selected from:
0.1 to 5.0 wt.%, like in range of 0.3 to 4.0 wt.% or 0.5 to 3.5 wt.%, of Me¹(I)₂O,
0.1 to 12 wt.%, like in a range of 0.5 to 11 wt.% or 1.0 to 9.5 wt.%, of Me²(II)O,
0.1 to 5.0 wt.%, like in a range of 0.1 to 4.5 wt.% or 0.1 to 4.0 wt.%, of Me³(III)₂O₃,
0.1 to 8.0 wt.%, like in a range of 1.0 to 7.0 wt.% or 2.0 to 6.5 wt.%, of Me⁴(IV)O₂,
0.02 to 6.0 wt.%, like in a range of 0.1 to 5.5 wt.% or 1.0 to 5.0 wt.%, of Me⁵(V)₂O₅,
0.02 to 1.0 wt.%, like in a range of 0.05 to 0.5 wt.% or 0.1 to 0.3 wt.%, mixed valence metal oxide,
0.1 to 1.5 wt.%, like in a range of 0.2 to 1.0 wt.% or 0.2 to 0.5 wt.%, of F,
wherein
Me¹(I)₂O is selected from one valent metal oxides (Me¹(I)₂O) and mixtures thereof, wherein Li₂O is excluded,
Me²(II)O is selected from two valent metal oxides and mixtures thereof,
Me³(III)₂O₃ is selected from three valent metal oxides and mixtures thereof, wherein Al₂O₃ is excluded,
Me⁴(IV)O₂ is selected from four valent metal oxides and mixtures thereof, wherein SiOz is excluded,
Me⁵(V)₂O₅ is selected from five valent metal oxides and mixtures thereof, and
Me⁶(VI)O₆ is selected from six valent metal oxides and mixtures thereof.

In many embodiments, the lithium alumosilicate glass-ceramic additionally comprises 0.1 to 7.0 wt.%, like in a range of 0.1 to 6.5 wt.% or 0.5 to 6.0 wt.%, of Me⁴(IV)O₂.

For example, the lithium alumosilicate glass-ceramic may comprise one or more additional components selected from:
0.0 to 5.0 wt.%, like in a range of 0.0 to 4.0 wt.% or 0.0 to 3.0 wt.%, of Na₂O,
0.0 to 5.0 wt.%, like in a range of 0.0 to 3.5 wt.% or 0.0 to 2.5 wt.%, of K₂O,
0.0 to 6.0 wt.%, like in a range of 0.0 to 5.0 wt.% or 0.0 to 4.5 wt.%, of MgO,
0.0 to 7.0 wt.%, like in a range of 0.0 to 5.0 wt.% or 0.0 to 5.5 wt.%, of CaO,
0.0 to 11.0 wt.%, like in a range of 0.0 to 10.0 wt.% or 0.0 to 9.5 wt.%, of SrO,
0.0 to 9.0 wt.%, like in a range of 0.0 to 8.0 wt.% or 0.0 to 7.5 wt.%, of ZnO,
0.0 to 5.0 wt.%, like in a range of 0.0 to 4.0 wt.% or 0.0 to 3.5 wt.%, of La₂O₃,
0.0 to 5.0 wt.%, like in a range of 0.0 to 4.0 wt.% or 0.0 to 3.5 wt.%, of Y₂O₃,
0.0 to 0.8 wt.%, like in a range of 0.0 to 0.5 wt.% or 0.0 to 0.3 wt.%, of Er₂O₃,
0.0 to 4.0 wt.%, like in a range of 0.0 to 3.0 wt.% or 0.0 to 2.5 wt.%, of Gd₂O₃,
0.0 to 2.0 wt.%, like in a range of 0.0 to 1.5 wt.% or 0.0 to 1.2 wt.%, of Eu₂O₃,
0.0 to 4.0 wt.%, like in a range of 0.0 to 3.0 wt.% or 0.0 to 2.5 wt.%, of Dy₂O₃,
0.0 to 8.0 wt.%, like in a range of 0.0 to 7.0 wt.% or 0.0 to 6.5 wt.%, of ZrO₂,
0.0 to 3.0 wt.%, like in a range of 0.0 to 2.0 wt.% or 0.0 to 1.5 wt.%, of SnO₂,
0.0 to 5.0 wt.%, like in a range of 0.0 to 4.0 wt.% or 0.0 to 3.5 wt.%, of CeO₂,
0.0 to 0.6 wt.%, like in a range of 0.0 to 0.4 wt.% or 0.0 to 0.2 wt.%, of V₂O₅,
0.0 to 7.0 wt.%, like in a range of 0.0 to 6.0 wt.% or 0.0 to 5.5 wt.%, of Ta₂O₅,
0.0 to 7.0 wt.%, like in a range of 0.0 to 6.0 wt.% or 0.0 to 5.5 wt.%, of Nb₂O₅,
0.0 to 0.6 wt.%, like in a range of 0.0 to 0.4 wt.% or 0.0 to 0.25 wt.%, of Tb₄O₇.

In many embodiments, the lithium alumosilicate glass-ceramic comprises one or more additional components selected from:
0.1 to 5.0 wt.%, like in a range of 0.3 to 4.0 wt.% or 0.5 to 3.0 wt.%, of Na₂O,
0.1 to 5.0 wt.%, like in a range of 0.3 to 3.5 wt.% or 0.3 to 2.5 wt.%, of K₂O,
0.1 to 6.0 wt.%, like in a range of 0.1 to 5.0 wt.% or 0.3 to 4.5 wt.%, of MgO,
0.1 to 7.0 wt.%, like in a range of 1.0 to 5.0 wt.% or 3.0 to 5.5 wt.%, of CaO,
0.1 to 11.0 wt.%, like in a range of 1.0 to 10.0 wt.% or 4.0 to 9.5 wt.%, of SrO,
0.1 to 9.0 wt.%, like in a range of 0.5 to 8.0 wt.% or 0.5 to 7.5 wt.%, of ZnO,
0.1 to 5.0 wt.%, like in a range of 0.5 to 4.0 wt.% or 1.0 to 3.5 wt.%, of LazOs,
0.1 to 5.0 wt.%, like in a range of 0.5 to 4.0 wt.% or 1.0 to 3.5 wt.%, of Y₂O₃,
0.1 to 0.8 wt.%, like in a range of 0.1 to 0.5 wt.% or 0.1 to 0.3 wt.%, of Er₂O₃,
0.1 to 4.0 wt.%, like in a range of 0.1 to 3.0 wt.% or 0.5 to 2.5 wt.%, of Gd₂O₃,
0.1 to 2.0 wt.%, like in a range of 0.1 to 1.5 wt.% or 0.1 to 1.2 wt.%, of Eu₂O₃,
0.1 to 4.0 wt.%, like in a range of 0.1 to 3.0 wt.% or 0.5 to 2.5 wt.%, of DyzOs,
0.1 to 7.0 wt.%, like in a range of 0.1 to 6.5 wt.% or 0.1 to 6.0 wt.%, of ZrO₂,
0.1 to 3.0 wt.%, like in a range of 0.1 to 2.0 wt.% or 0.5 to 1.5 wt.%, of SnO₂,
0.1 to 5.0 wt.%, like in a range of 0.1 to 4.0 wt.% or 0.1 to 3.5 wt.%, of CeO₂,
0.1 to 0.6 wt.%, like in a range of 0.1 to 0.4 wt.% or 0.1 to 0.2 wt.%, of V₂O₅,
0.1 to 7.0 wt.%, like in a range of 0.5 to 6.0 wt.% or 1.0 to 5.5 wt.%, of Ta₂O₅,
0.1 to 7.0 wt.%, like in a range of 0.5 to 6.0 wt.% or 1.0 to 5.5 wt.%, of Nb₂O₅,
0.1 to 0.6 wt.%, like in a range of 0.1 to 0.4 wt.% or 0.1 to 0.25 wt.%, of Tb₄O₇.

In many embodiments, the lithium alumosilicate glass-ceramic additionally comprises 0.1 to 7.0 wt.%, like in a range of 0.1 to 6.5 wt.% or 0.5 to 6.0 wt.%, of ZrOz. The weight amount of ZrOz in the lithium alumosilicate glass-ceramic may influence its opacity (contrast ratio), wherein a higher weight amount may lead to a higher opacity (contrast ratio).

In certain embodiment, the lithium alumosilicate glass-ceramic comprises:
62 to 74 wt.%, like in a range of 64 to 72 wt.% or 66 to 70 wt.%, of SiO₂,
4.0 to 10 wt.% of Li₂O,
4.0 to 9.0 wt.%, like in a range of 5.0 to 9.0 wt.% or 6.0 to 8.0 wt.%, of Al₂O₃, and
a nucleating agent (typically P₂O₅).

In certain embodiment, the lithium alumosilicate glass-ceramic comprises:
62 to 74 wt.%, like in a range of 64 to 72 wt.% or 66 to 70 wt.%, of SiO₂,
4.0 to 10 wt.% of Li₂O,
4.0 to 9.0 wt.%, like in a range of 5.0 to 9.0 wt.% or 6.0 to 8.0 wt.%, of Al₂O₃,
1.0 to 6.0 wt.%, like in a range of 2.0 to 5.0 wt.% or 3.0 to 4.0 wt.%, a nucleating agent (typically P₂O₅), and optionally
0.1 to 5.0 wt.%, like in range of 0.3 to 4.0 wt.% or 0.5 to 3.5 wt.%, of Me¹(I)₂O (e.g., Na₂O and K₂O),
0.1 to 5.0 wt.%, like in a range of 0.5 to 4.0 wt.% or 1.0 to 3.0 wt.%, of Me²(II)O (e.g., ZnO and MgO),
0.0 to 0.1 wt.%, like 0.1 wt.%, of Me³(III)₂O₃ (e.g., Er₂O₃),
0.1 to 6.0 wt.%, like in a range of 1.0 to 6.0 wt.% or 2.0 to 4.0 wt.%, of Me⁴(IV)O₂ (e.g., ZrO₂ and CeO₂),
0.1 to 0.5 wt.%, like in a range of 0.1 to 0.4 wt.% or 0.1 to 0.3 wt.%, of Me⁵(V)₂O₅ (e.g., V₂O₅), and
0.1 to 0.5 wt.%, like in a range of 0.1 to 0.4 wt.% or 0.1 to 0.3 wt.%, mixed valence metal oxide (e.g., Tb₄O₇).

In one embodiment, the lithium alumosilicate glass-ceramic comprises the keatite s.s. crystal phase as a main crystal phase, wherein the lithium alumosilicate glass-ceramic comprises:
62 to 76 wt.%, like in a range of 64 to 74 wt.%, of SiO₂,
7.0 to 18 wt.%, like in a range of 8.5 to 16 wt.%, of Li₂O,
4.0 to 15 wt.%, like in a range of 5.0 to 15 wt.%, of Al₂O3.

In one embodiment, the lithium alumosilicate glass-ceramic comprises Li₂Si₂O₅ as the main crystal phase and the keatite s.s. crystal phase as a minor crystal phase, and comprises:
wherein the lithium alumosilicate glass-ceramic comprises:
64 to 78 wt.%, like in a range of 66 to 76 wt.%, of SiO₂,
11 to 20 wt.%, like in a range of 13 to 18 wt.%, of Li₂O,
2.0 to 9.0 wt.%, like in a range of 2.5 to 7.5 wt.%, of Al₂O3.

In one embodiment, the lithium alumosilicate glass-ceramic comprises a quartz s.s. crystal phase as the main crystal phase and the keatite s.s. crystal phase as a minor crystal phase, and wherein the lithium alumosilicate glass-ceramic comprises:
wherein the lithium alumosilicate glass-ceramic comprises:
60 to 78 wt.%, like in a range of 62 to 78 wt.%, of SiO₂,
8.5 to 17 wt.%, like in a range of 8.5 to 15 wt.%, of Li₂O,
2.0 to 13 wt.%, like in a range of 3.5 to 11 wt.%, of Al₂O₃.

In one embodiment, the lithium alumosilicate glass-ceramic comprises a Li₂SiO₃ crystal phase as the main crystal phase and the keatite s.s. crystal phase as a minor crystal phase, and wherein the lithium alumosilicate glass-ceramic comprises:
wherein the lithium alumosilicate glass-ceramic comprises:
60 to 68 wt.%, like in a range of 61 to 67 wt.%, of SiO₂,
18 to 24 wt.%, like in a range of 19 to 24 wt.%, of Li₂O,
2.0 to 7.5 wt.%, like in a range of 4.0 to 6.5 wt.%, of Al₂O₃.

The lithium alumosilicate glass-ceramic is typically colored. When the lithium alumosilicate glass-ceramic is colored, it typically comprises colorants (e.g., fluorescent or non-fluorescent pigments, coloring metal oxides, and the like) in an amount in a range of 0.1 wt.% to equal to or less than 1 wt.%. The lithium alumosilicate glass-ceramic may have a color that matches a shade of the VITA classical A1-D4^{®} shade guide with VITA Bleached Shades manufactured by Vita Zahnfabrik. The shade may be, but is not limited to, A1, A2, A3.5, A4, B1, B2, B3, B4, C1, C2, C3, C4, D1, D2, D3, D4, BL1 or BL2.

### 3. Structure and shape

The structure and shape of the lithium alumosilicate glass-ceramic is not particularly limited.

The lithium alumosilicate glass-ceramic may be a powder. The powder may be in form of a suspension which is suitable for use in an additive manufacturing process such as stereo lithography, inkjet printing, screen printing, powder bed printing or fused deposition modeling (fused filament fabrication). In one embodiment, the lithium alumosilicate glass-ceramic is provided in form of a suspension which is suitable for use in an additive manufacturing process, and preferably a stereolithography process. In said embodiment, the suspension typically comprises the lithium alumosilicate glass-ceramic, an organic monomer solution, and a photo initiator. When the lithium alumosilicate glass-ceramic is provided in form of a powder, it may be present in a partially crystallized form. This means that the lithium alumosilicate glass-ceramic powder may comprise a weight amount of the keatite s.s. crystal phase which is lower than a weight amount of the keatite s.s. crystal phase obtained after full crystallization (e.g., after full crystallization of a dental restoration prepared from the lithium alumosilicate glass-ceramic powder).

The lithium alumosilicate glass-ceramic may be a solid body, like a dental body. For example, the lithium alumosilicate glass-ceramic may be a dental blank, like a dental mill blank or a dental press blank, or it may be a dental restoration. It is also possible that the lithium alumosilicate glass-ceramic is a part of a dental body, like a part of a dental blank or a part of a dental restoration. For example, the lithium alumosilicate glass-ceramic may be a layer of the dental body.

In one aspect, the present invention provides a use of a lithium alumosilicate glass-ceramic of the present invention as a dental body or a part thereof, and preferably a dental body according to the present invention. The dental body is further described in the sections herein below, e.g., section III.

### 4. Process for preparation

The present invention is further directed to a process for preparing a lithium alumosilicate glass-ceramic comprising a keatite s.s. crystal phase, preferably a lithium alumosilicate glass-ceramic according to the present invention.

The lithium alumosilicate glass-ceramic may be prepared following different process routes, including process routes referred to herein as "powder route" and as "solid glass route".

### 4.1 Powder route

In one embodiment, the present invention provides a process for preparing a lithium alumosilicate glass-ceramic comprising a keatite s.s. crystal phase, preferably a lithium alumosilicate glass-ceramic according to the present invention, the process comprising the steps of:
- providing a powder selected from glass powders, glass-ceramic powders and mixtures thereof;
- preparing a shaped body from the powder; and
- subjecting the shaped body to a heat treatment.

### 4.1.1 Providing the powder

The process comprises the step of providing a powder selected from glass-powders, glass-ceramic powders, and mixtures thereof. The powder is preferably a powder of a glass or glass-ceramic of the present invention, for example, as described herein in section I. The powder may be a mixture of two or more powders of the glass or a glass-ceramic of the present invention.

The powder is typically provided starting by melting chemical starting materials which are suitable for preparing a glass. The starting materials may be melted at a temperature between 1300 to 1650°C (e.g., between 1450 to 1650°C) over a time period of 30 minutes to 10 h (e.g., between 30 minutes to 3 h). In many embodiments, the starting materials are melted at a temperature between 1475 to 1625°C, like 1500 to 1600°C, over a time period of between 45 minutes to 180 min, like between 60 to 120 min. The melt may be poured into water for freezing a glass frit. To increase homogeneity of the glass, the glass may be re-melted under the same or similar conditions and re-frozen in water. The obtained glass frit may be dried in an oven. The obtained glass frit is subsequently ground to obtain a powder. The volume-based median particle size d₅₀ of the powder may be in a range of 0.1 to 100 µm, like in a range of 1 to 50 µm. The volume-based top cut particle size d₉₈ of the powder may be below 90 µm, like below 63 µm. The powder obtained by melting, freezing and grinding a glass is typically a glass powder, i.e., is essentially free of a crystalline phase.

The powder may be combined with one or more additives such as, but not limited to, one or more pigments, one or more fluorescent pigments, one or more pressing aids, and/or one or more binders. Suitable pigments may be, but are not limited to, doped spinels, doped zirconium oxides, zirconium oxide, doped zirconium silicates, doped yttrium silicates or tin oxide. Suitable pressing aids may be, but are not limited, to polyethylene glycols or stearates. Suitable binders may be, but are not limited to, polyvinyl alcohols and cellulose derivatives, such as sodium carboxymethylcellulose.

The powder, optionally in combination with the one or more additives, may be provided in dry form. The powder together with the one or more additives may be provided in form of a dry blend. The dry blend may be a particulate mixture or may be in the form of granules. The granules may be prepared by granulating the powder together with the one or more additives, typically comprising one or more binders, in a granulation process as known in the art. The dry blend may comprise the powder and the one or more additives in weight amounts as described herein above, for example in section I.2, in connection with the glass or glass-ceramic according to the present invention.

Alternatively, the powder may be present in liquid form, and particularly in the form of a suspension. The further ingredients of the suspension may depend on the use of the suspension. For example, the suspension may be for use in die casting or slip casting. In such case, the suspension may be an aqueous suspension as described herein above, for example in section I.2, in connection with the glass or glass-ceramic according to the present invention.

### 4.2.1 Preparing the shaped body

The process further comprises the step of preparing a shaped body from the powder (e.g., the glass powder). The shaped body may be porous. The shaped body may be prepared by molding the powders in a suitable mold to obtain a molded body. The preparation of the molded body may differ depending on the form in which the powder is provided. When the powder is provided in dry form, e.g., in form of a dry blend with one or more additives, the powder may be introduced into the mold followed by a compacting step (e.g., a pressing step, like an uniaxial pressing). The compacting step may be a cold compacting step. The cold compacting (e.g., cold pressing) may be carried out at a temperature of less than 60°C, like in a range of 15 to 35°C, and at a pressure of 2 to 30 bar, like in a range of 5 to 15 bar. Additionally or alternatively, the compacting step may be a hot compacting step. The hot compacting step (e.g., hot pressing step) may be carried out at a temperature in a range of 650 to 850°C, like in the range of 700 to 800°C, and a pressure in a range of 5 to 50 MPa, preferably 10 to 30 MPa. The hot compacting is typically carried out over a time period in a range of 0.1 to 10 min, like in a range of 0.3 to 5 min. The hot compacting step may be carried at an atmospheric pressure below ambient pressure, for example, at an atmospheric pressure of less than 0.1 bar, like in a range of 0.01 to 0.8 bar. The hot compacting may be a pressure sintering. After the hot compacting, a compacted body may be obtained which is essentially non-porous.

When the powder is provided in liquid form, the powder may be introduced into the mold followed by removing the liquid. The mold may be a mold suitable for die casting or slip casting. The mold may have pores through which the liquid can be removed. Removal of the liquid may be carried out and/or supported by, e.g., pressing, suction, and/or freeze drying.

### 4.3.1 Heat treating the shaped body

The process further comprises the step of subjecting the shaped body (e.g., the molded body) to a heat treatment to obtain the lithium alumosilicate glass-ceramic.

The heat treatment typically comprises a sintering step. During the sintering step, the shaped (powder) body is densified by applying heat. The sintering step may be a conventional sintering step which is carried out without applying additional pressure. It is however also possible that the sintering step is a pressure sintering. In pressure sintering, the densification of the shaped (powder) body is assisted by applying pressure. The pressure sintering may be carried simultaneously with the hot compacting step as described above.

The heat treatment comprises a crystallization step for forming a keatite s.s. crystal phase. In the crystallization step, one or more new crystal phases (including the keatite s.s. crystal phase) may be created, or one or more existing crystal phases may be converted to another crystal phase (like into the keatite s.s. crystal phase). The crystallization step may be carried out together with the sintering step or separately, like after the sintering step.

The heat treatment may be carried out at a maximum temperature in a range of 750 to 1050 °C, like in a range of 800 to 1020°C, like in a range of 840 to 1000°C, like in a range of 850 to 1000°C, or in a range of 880 to 980°C. The maximum temperature may be hold for a time period in a range of 2 minutes to 180 min, like in a range of 5 minutes to 120 min, like in a range of 10 minutes to 90 minutes.

The heat treatment may comprise one or more heating steps, like two, three or more heating steps. The different heating steps may be used for different purposes. For example, one or more heating steps may be included for debinding a binder which is present in the shaped body (e.g., at a temperature of less than 500°C), for nucleating glass at a temperature below a maximum temperature, and/or for heating (sintering) to a maximum temperature.

For example, the heat treatment may comprise a first heating and a second heating step. The first heating step may end at a temperature in a range of 450 to 700°C, like in a range of 480 to 650°C or 500 to 600°C. An end temperature of the first heating step may be hold for a time period in a range of 5 minutes to 120 min, like in a range of 10 minutes to 60 min, like in a range of 15 minutes to 45 min, e.g., for about 30 min. The first heating step may have a heating rate in a range of 5 to 15 K/min. The second heating step may start at the end temperature of the first heating step and may end at the maximum temperature, e.g., the maximum temperature as described herein above. The second heating step may have a heating rate in a range of 5 to 15 K/min.

The heat treatment may partially or fully be carried out at a pressure below ambient pressure. For the present disclosure, ambient pressure is defined as 1.013 bar. For example, the heat treatment may partially or fully be carried out at a pressure of less than 0.9 bar, like in a range of 10 to 300 mbar or 30 to 120 mbar. The heat treatment is typically followed by a cooling step. The cooling step starts at the maximum temperature and ends at a temperature which is suitable for handling the lithium alumosilicate glass-ceramic (like removing the glass-ceramic from a sintering furnace).

The heat treating step provides the lithium alumosilicate glass-ceramic. The lithium alumosilicate glass-ceramic is obtained in form of a solid body.

The process may comprise one or more additional process steps which are typical in the art, and which may be carried out before, between or after the process steps described herein. Additional steps may be, but are not limited to, adjusting the particle size distribution of the powder (e.g., by sieving) and/or preparing the surface of the shaped body (e.g., the molded body) and/or the surface of the lithium alumosilicate glass-ceramic (e.g., by grinding, lapping or polishing).

According to one preferred embodiment, the process for preparing the lithium alumosilicate glass-ceramic comprises the steps of:
- providing a glass powder, and preferably a glass powder according to the present invention, wherein the glass powder is provided in form of granules at least comprising the glass powder, one or more binders, and one or more pigments,
- preparing a shaped body from the glass powder,
- heat treating the shaped body to obtain the lithium alumosilicate glass-ceramic.

### 4.2 Solid glass route

In one embodiment, the present invention provides a process for preparing a lithium alumosilicate glass-ceramic comprising a keatite s.s. crystal phase, preferably a lithium alumosilicate glass-ceramic according to the present invention, the process comprising the steps of:
- preparing a solid glass body from a molten glass; and
- subjecting the solid glass body to a heat treatment.

The process comprises the step of providing a solid glass body, and preferably a solid glass body according to the present invention. The solid glass body is typically provided starting by melting chemical starting materials which are suitable for preparing the glass. The starting materials may be melted at a temperature between 1300 to 1650°C (e.g., between 1450 to 1650°C) over a time period of 30 minutes to 10 h (e.g., between 30 minutes to 3 h). In several embodiments, the starting materials are melted at a temperature between 1400 to 1600°C, like 1450 to 1550°C (e.g., about 1500°C), over a time period of between 45 minutes to 180 min, like between 60 to 180 minutes (e.g., about 120 min). The melt may be poured into a mold in which the molten glass solidifies to the solid glass body.

The process further comprises the step of subjecting the solid glass body to a heat treatment. The heat treatment comprises a crystallization step for forming a keatite s.s. crystal phase. The heat treatment may be carried out at a maximum temperature in a range of 750 to 1050 °C, like in a range of 800 to 900°C, like at about 850°C. The maximum temperature may be hold for a time period in a range of 2 minutes to 180 min, like in a range of 2 minutes to 30 min, like in a range of 5 minutes to 20 minutes, and for example for about 10 minutes.

The heat treatment may comprise one or more heating steps, like two, three or more heating steps. The different heating steps may be used for different purposes. For example, a first heating step may be used for nucleating the glass, for example, at a temperature in a range of 450 to 550°C, like about 500°C. An end temperature of the first heating step may be hold for a time period in a range of 5 minutes to 30 min, like about 10 minutes. The first heating step may have a heating rate in a range of 5 to 15 K/min. The second heating step may start at the end temperature of the first heating step and may end at a maximum temperature in a range of 750 to 1050 °C, like in a range of 800 to 900°C, like at about 850°C. The second heating step may have a heating rate in a range of 5 to 15 K/min. The heating steps may be carried out successively in one single heat treatment.

Alternatively, it is possible that the heat treatment comprises two or more separate heat treatments, like two separate heat treatments. For example, it is possible that the heat treatment comprises a first heat treatment and a second heat treatment. In the first heat treatment, the solid glass body is (partially) crystallized to a solid glass-ceramic body which is essentially free of a keatite s.s. crystal phase. The solid-glass ceramic body may comprise a lithium metasilicate crystal phase, e.g., in form of a main crystal phase. The first heat treatment may comprise one or more heating steps. For example, the first heat treatment may comprise a first heating step for nucleating the glass, for example, at a temperature in a range of 450 to 550°C, like about 500°C, which may be held for about 10 minutes. The first heat treatment may further comprise a second heating step which may end at a maximum temperature of in range of 650 to 750°C, like about 700°C. The second heating step may be a heating step for crystallizing lithium metasilicate. The first heat treatment provides a solid glass-ceramic body which may be further processed, e.g., by a CAD/CAM process, to provide a precursor of a dental restoration. Thus, the further processing, e.g., by the CAD/CAM process, may be carried out between the first heat treatment and the second heat treatment. After the first heat treatment, the (optionally further processed) solid glass-ceramic body is subjected to a second heat treatment to provide the lithium alumosilicate glass-ceramic according to the invention. In the second heat treatment, a keatite s.s. crystal phase is formed, optionally in combination with a Li₂Si₂O₅ crystal phase, and further optionally one or more additional crystal phases. The second heat treatment may comprise a heating step at a maximum temperature in a range of 750 to 1050 °C, like in a range of 800 to 900°C, like at about 850°C. The maximum temperature may be hold for a time period in a range of 2 minutes to 180 min, like in a range of 2 minutes to 30 min, like in a range of 5 minutes to 20 minutes, and for example for about 10 minutes. The second heating step may have a heating rate in a range of 5 to 15 K/min.

The process may comprise one or more additional process steps which are typical in the art, and which may be carried out before, between or after the process steps described herein. Additional steps may be, but are not limited to, preparing surface of the lithium alumosilicate glass-ceramic (e.g., by grinding, lapping or polishing).

### III. The dental body

In one aspect, the present invention provides a dental body comprising a glass-ceramic body comprising the lithium alumosilicate glass-ceramic according to the present invention.

### 1. Internal structure of the glass-ceramic body

The dental body comprises a glass-ceramic body. The glass-ceramic body comprises the lithium alumosilicate glass-ceramic according to the present invention. The glass-ceramic body may be partially or entirely composed of the lithium alumosilicate glass-ceramic of the present invention. The glass-ceramic body may comprise one or more types of the lithium alumosilicate glass-ceramic.

The glass-ceramic body of the dental body may have a specific internal structure. In one embodiment, the glass-ceramic body has a substantially homogenous internal structure. In such case, the glass-ceramic body is entirely composed of a single type of the lithium alumosilicate glass-ceramic. Thereby, a dental body may be provided having a glass-ceramic body with substantially homogenous properties, like optical, mechanical, and/or thermal properties.

It is however also possible, and often preferred, that the glass-ceramic body of the dental body has a multilayer structure or a gradient structure. A "multilayer structure" as used herein means that the glass-ceramic body has structure comprising two or more distinct layers. A "gradient structure" as used herein means that the glass-ceramic body comprises at least one section in which the weight amount of the glass-ceramic bulk material gradually changes in a direction of the glass-ceramic body. "Gradual change" as used herein encompasses a linear gradual change and a non-linear gradual change. A section of the glass-ceramic body with a gradient structure differs from a section with a multilayer structure in that it does not contain distinct layers.

The glass-ceramic body may comprise two or more layers (like 2 to 10 layers, like 2 to 8 layers, 2 to 6 layers, 2 to 4 layers, 3 layers or 2 layers) or three or more layers (like 3 to 10 layers, like 3 to 8 layers, 3 to 6 layers, 4 layers, 3 layers). The layers are different from one another. The layers may differ in terms of their chemical composition (e.g., at least one chemical component may differ in an amount of at least 0.5 wt.% or at least 1.0 wt.%), their crystal phases (e.g., at least one crystal phase may differ in an amount of at least 1.0 wt.%), or both. The layers may differ in their color. It is to be understood that, when the glass-ceramic body comprises two or more layers (like 2 to 10 layers, like 2 to 8 layers, 2 to 6 layers, 2 to 4 layers, 3 layers or 2 layers) of the lithium alumosilicate glass-ceramic according to the present invention, the two or more layers are composed of different types (embodiments) of the lithium alumosilicate glass-ceramic according to the present invention. The two or more layers of the glass-ceramic body are not particularly limited in terms of their dimensions and shapes. One or more layers may be non-planar or substantially planar. In one embodiment, the layers are substantially planar. In that context, "substantially planar" means that a layer is planar, subject to a tolerance of 5% of an average thickness of the layer. The layers of the glass-ceramic body may be arranged such that the boundaries of the layers are substantially parallel to each other.

When the glass-ceramic body has a multilayer structure, the layers of the glass-ceramic body comprise at least one layer of the lithium alumosilicate glass-ceramic according to the present invention, like 1 to 8 layers, 1 to 6 layers, 1 to 4 layers, 1 to 3 layers, like two layers or one layer. When the glass-ceramic body comprises only one single layer of the lithium alumosilicate glass-ceramic according to the present invention, it further comprises one or more layers of a different glass-ceramic material which is suitable for use in a dental body but which does not contain a keatite s.s. crystal phase. When the glass-ceramic body comprises two or more layers of the lithium alumosilicate glass-ceramic according to the present invention (e.g., 2 to 8 layers, 2 to 6 layers, 2 to 4 layers, 3 layers, or 2 layers), the two or more layers are arranged on top of each other, and typically in an adjacent manner. The two or more layers of the lithium alumosilicate glass-ceramic according to the present invention may be combined with a layer of a different glass-ceramic material which does not contain a keatite s.s. crystal phase, wherein the layer of the different glass-ceramic material is arranged at the top and/or end of the stacked two or more layers. In one embodiment, the glass-ceramic body is composed of two or more layers of the lithium alumosilicate glass-ceramic according to the present invention (e.g., 2 to 8 layers, 2 to 6 layers, 2 to 4 layers, 3 layers, or 2 layers) or of three or more layers of the lithium alumosilicate glass-ceramic according to the present invention (e.g., 3 to 8 layers, 3 to 6 layers, 4 layers, or 3 layers).

The two or more layers or three or more layers of the glass-ceramic body may differ in a weight amount of the keatite s.s. crystal phase. This includes that one of the layers may not contain a keatite s.s. crystal phase, i.e., one of the layers may be composed of different glass-ceramic material not containing a keatite s.s. crystal phase. In one embodiment, the glass-ceramic body comprises three or more layers (e.g., 3 to 8 layers, 3 to 6 layers, 4 layers, or 3 layers), wherein the three or more layers differ in their weight amount of the keatite s.s. crystal phase. The weight amount of the keatite s.s. crystal phase in the three or more layers may differ such that a weight gradient of the keatite s.s. crystal phase is formed in a stacking direction of the layers. The weight amount of the keatite s.s. crystal phase is determined on the basis of the total weight of the respective layer of the glass-ceramic body.

When the glass-ceramic body of the dental body contains different layers having different weight amounts of a keatite s.s. crystal phase, the properties of the glass-ceramic body of the dental body change in a direction of the stacking order of the layers. For example, the translucency of the glass-ceramic body typically increases and/or the coefficient of the thermal expansion typically decreases, when the weight amount of the keatite s.s. crystal phase increases in a layer.

The layers of the glass-ceramic body are typically colored. The layers of the glass-ceramic body may differ in their color. The layers of the glass-ceramic body may differ in their color and in their weight amount of the keatite s.s. crystal phase. Thereby, it is possible to provide the dental body with a desirable color and translucency, and particularly with a color and translucency which changes across the dental body in a way to resemble the natural appearance of a person's tooth. The glass-ceramic body may have a color that matches a shade of by the VITA classical A1-D4^{®} shade guide with VITA Bleached Shades manufactured by Vita Zahnfabrik. The shade may be, but is not limited to, A1, A2, A3.5, A4, B1, B2, B3, B4, C1, C2, C3, C4, D1, D2, D3, D4, BL1 or BL2.

In one embodiment, the glass-ceramic body comprises three or more layers (e.g., 3 to 8 layers, 3 to 6 layers, 4 layers, or 3 layers), wherein two or more (e.g., 2 to 8, 2 to 6 layers, 4 layers, or 3 layers) of the three or more layers are layers of different types of the lithium alumosilicate glass-ceramic according to the present invention, and wherein the three or more layers differ in their weight amount of the keatite s.s. crystal phase, and optionally in their color. The weight amount of the keatite s.s. crystal phase in the three or more layers may differ such that a weight gradient of the keatite s.s. crystal phase is formed in a stacking direction of the layers.

In several embodiments, the (colored) glass-ceramic body comprises (or is composed of):
a top layer,
optionally at least one intermediate layer (e.g., 1 to 6 intermediate layers, 1 to 4 intermediate layers, 2 intermediate layers, or 1 intermediate layer), and
a bottom layer,
wherein the top layer and the optional at least one intermediate layer (if present) are layers of different types of the lithium alumosilicate glass-ceramic of the present invention, and the bottom layer is either a layer of another different type of a lithium alumosilicate glass-ceramic of the present invention or a layer of a different glass-ceramic material not containing a keatite s.s. crystal phase, and
wherein the top layer, the optional at least one intermediate layer, and the bottom layer differ in their weight amount of a keatite s.s. crystal phase.

The top layer typically has a higher weight amount of the keatite s.s. crystal phase than the bottom layer. The top layer, the optional at least one intermediate layer, and the bottom layer typically differ in their weight amounts of the keatite s.s. crystal phase such that the weight amount of the keatite s.s. crystal phase increases from the bottom layer to the top layer. When the optional at least one intermediate layer is present, the weight amount of the keatite s.s. crystal phase typically increases from the bottom layer to the at least one intermediate layer, and from the at least one intermediate layer to the top layer. The weight amount of the keatite s.s. crystal phase may increase layer-by-layer from the bottom layer to the top layer. The top layer, the optional at least one intermediate layer, and the bottom layer may additionally differ in their colors.

In certain embodiments, the (colored) glass-ceramic body comprises (or is composed of):
a top layer,
at least one intermediate layer (e.g., 1 to 6 intermediate layers, 1 to 4 intermediate layers, 2 intermediate layers, or 1 intermediate layer), and
a bottom layer,
wherein the top layer and the at least one intermediate layer are layers of different types of a lithium alumosilicate glass-ceramic of the present invention, and the bottom layer is either a layer of another different type of a lithium alumosilicate glass-ceramic of the present invention or a layer of a different glass-ceramic material not containing a keatite s.s. crystal phase, and
wherein the top layer, the at least one intermediate layer, and the bottom layer differ in their weight amount of a keatite s.s. crystal phase such that the weight amount of the keatite s.s. crystal phase increases (optionally layer-by-layer) from the bottom layer to the at least one intermediate layer, and from the at least one intermediate layer to the top layer.

In certain embodiments, the bottom layer is a layer of the lithium alumosilicate glass-ceramic of the present invention. In certain embodiments, the (colored) glass-ceramic body comprises (or is composed of):
a top layer,
optionally at least one intermediate layer (e.g., 1 to 6 intermediate layers, 1 to 4 intermediate layers, 2 intermediate layers, or 1 intermediate layer), and
a bottom layer,
wherein the top layer, the optional at least one intermediate layer, and the bottom layer are layers of different types of a lithium alumosilicate glass-ceramic of the present invention which differ in their weight amount of a keatite s.s. crystal phase, and typically such that the weight amount of the keatite s.s. crystal phase increases (optionally layer-by-layer) from the bottom layer to the optional at least one intermediate layer, and from the at least one intermediate layer to the top layer.

In embodiments, the (colored) glass-ceramic body comprises (or is composed of):
a top layer,
at least one intermediate layer (e.g., 1 to 6 intermediate layers, 1 to 4 intermediate layers, 2 intermediate layers, or 1 intermediate layer), and
a bottom layer,
wherein the top layer, the optional at least one intermediate layer, and the bottom layer are layers of different types of a lithium alumosilicate glass-ceramic of the present invention which differ in their weight amount of a keatite s.s. crystal phase, and typically such that the weight amount of the keatite s.s. crystal phase increases (optionally layer-by-layer) from the bottom layer to the at least one intermediate layer, and from the at least one intermediate layer to the top layer.

In an alternative embodiment, the glass-ceramic body has a gradient structure. The glass-ceramic body may comprise a lithium alumosilicate glass-ceramic according to the present invention in combination with one or more different glass-ceramic materials. The one or more different glass-ceramic materials may be a different type (embodiment) of the lithium alumosilicate glass-ceramic according to the present invention, or another glass-ceramic material which is suitable for use in a dental body and which does not contain a keatite s.s. crystal phase. The weight amount of a lithium alumosilicate glass-ceramic according to the present invention may gradually change in a direction of the glass-ceramic body. In such case, the weight amount of the keatite s.s. crystal phase in the glass-ceramic body, and optionally its color, may gradually change in a direction of the glass-ceramic body. This includes that the weight amount of the keatite s.s. crystal phase in the glass-ceramic body changes such that a section of the glass-ceramic body may contain no keatite s.s. crystal phase. The glass-ceramic body may be composed of at least two types (embodiments) of the lithium alumosilicate glass-ceramic of the present invention, wherein the weight amounts of the at least two types lithium alumosilicate glass-ceramic gradually change in a direction of the glass-ceramic body.

In certain embodiments, the (colored) glass-ceramic body comprises (or is composed of):
a top section,
an intermediate section, and
a bottom section,
wherein the top section is entirely composed of one type of a lithium alumosilicate glass-ceramic according to the present invention, and the bottom section is entirely composed of a different type of a lithium alumosilicate glass-ceramic according to the present invention or of another glass-ceramic material not containing a keatite s.s. crystal phase, and wherein the weight amount of the lithium alumosilicate glass-ceramic according of the top section gradually changes across the intermediate section in a direction to the bottom layer.

The lithium alumosilicate glass-ceramic of the top section typically has a higher weight amount of the keatite s.s. crystal phase than the lithium alumosilicate glass-ceramic of the bottom section or the other glass-ceramic of the bottom section. The weight amount of the lithium alumosilicate glass-ceramic according of the top section may gradually decrease across the intermediate section in a direction to the bottom layer such that a weight amount of the keatite s.s. crystal phase gradually decreases across the intermediate section in a direction from the top section to the bottom section. When the glass-ceramic body is colored, the color may gradually change across the intermediate section in a direction from the top section to the bottom section.

In certain embodiments, the (colored) glass-ceramic body comprises (or is composed of):
a top section,
an intermediate section, and
a bottom section,
wherein the top section is entirely composed of one type of a lithium alumosilicate glass-ceramic according to the present invention, and the bottom section is entirely composed of a different type of a lithium alumosilicate glass-ceramic according to the present invention, and wherein the weight amount of the lithium alumosilicate glass-ceramic according of the top section gradually decreases across the intermediate section in a direction to the bottom layer such that a weight amount of the keatite s.s. crystal phase gradually decreases across the intermediate section in a direction from the top section to the bottom section.

The glass-ceramic body of the dental body may be characterized by a gradient of a specific property. The glass-ceramic body of the dental body may be characterized by a translucency gradient. In such case, the translucency may change stepwise (e.g., when the glass-ceramic body has a multilayer structure) or gradually (e.g., when the glass-ceramic body has a gradient structure) in a direction of the glass-ceramic body. Additionally or alternatively, the glass-ceramic body of the dental body may be characterized by a color gradient. In such case, the color may change stepwise (e.g., when the glass-ceramic body has a multilayer structure) or gradually (e.g., when the glass-ceramic body has a gradient structure) in a direction of the glass-ceramic body. The translucency gradient and/or color gradient may be determined by preparing (e.g., by milling or sawing) different test bodies from the glass-ceramic body, e.g., a test body for each layer or a test body for each section, and measuring the test bodies for their contrast ratio (opacity) and/or color using the methods as described herein.

Additionally or alternatively, the glass-ceramic body of the dental body may be characterized by a gradient of the coefficient of thermal expansion. In such case, the coefficient of thermal expansion may change stepwise (e.g., when the glass-ceramic body has a multilayer structure) or gradually (e.g., when the glass-ceramic body has a gradient structure) in a direction of the glass-ceramic body. The gradient of the coefficient of thermal expansion may be determined by preparing (e.g., by milling or sawing) different test bodies from the glass-ceramic body, e.g., a test body for each layer or a test body for each section, and measuring the test bodies for coefficient of thermal expansion using the method as described herein.

### 2. Type of dental body and shape

The dental body may be a dental blank, like a dental mill blank or a dental press blank, which is suitable for preparing a dental restoration. In one preferred embodiment, the dental body is a dental mill blank. The dental blank is not particularly limited as regards its shape or its dimensions as long as it is suitable for use for preparing a dental restoration (e.g., using a CAD/CAM process or using hot pressing into a mold). The dental blank may have, but is not limited to, the form of a rectangular block, an ingot, a disc, a cylinder, a dental preform (e.g., an abutment preform or a tooth sector), a cone, a cone segment, a pyramid, or a pyramid segment. The dental blank may comprise one or more additional components in addition to its glass-ceramic body, like one or more additional components which are typically part of a commercially available dental blank. For example, when the dental body is a dental blank (e.g., a dental mill blank), the one or more additional components may be one or more outside layers, like protective layers or printing layers (e.g., containing product information, a bar code or a QR code), or a holding pin, like a holding pin for holding the dental mill blank in a device when machining it, e.g., using a CAD/CAM process processing. The glass-ceramic body of the dental blank may be colored. The glass-ceramic body of the dental blank may have a color that matches a shade of the VITA classical A1-D4^{®} shade guide with VITA Bleached Shades manufactured by Vita Zahnfabrik. The shade may be, but is not limited to, A1, A2, A3.5, A4, B1, B2, B3, B4, C1, C2, C3, C4, D1, D2, D3, D4, BL1 or BL2.

In several embodiments, the dental body is a dental blank, and particularly a dental mill blank, wherein the dental blank is composed of the (colored) glass-ceramic body, and one or more additional components, like one or more outside layers, like protective layers or printing layers (e.g., containing product information, a bar code or a QR code), or a holding pin.

In several embodiments, the dental body is a dental blank, and particularly a dental mill blank, comprising a (colored) glass-ceramic body having a multilayer structure or a gradient structure, and preferably a multilayer structure, as described herein, e.g., in section III.1.

In one embodiment, the dental body is a dental blank comprising a (colored) glass-ceramic body comprising (or being composed of):
a top layer as described herein above, e.g., in section III.1, for preparing an incisal or occlusal zone, or a part thereof, in a dental restoration;
optionally at least one intermediate layer as described herein above, e.g., in section III.1, for preparing a transition zone, or a part thereof, in a dental restoration;
a bottom layer as described herein above, e.g., in section III.1, for preparing a dentin zone, or a part thereof, of a dental restoration.

In one embodiment, the dental body is a dental blank comprising a (colored) glass-ceramic body comprising (or being composed of):
a top section as described herein above, e.g., in section III.1, for preparing an incisal or occlusal zone, or a part thereof, in a dental restoration;
an intermediate section as described herein above, e.g., in section III.1, for preparing a transition zone, or a part thereof, in a dental restoration;
a bottom section as described herein above, e.g., in section III.1, for preparing a dentin zone, or a part thereof, of a dental restoration.

The dental body may be a dental restoration. When the dental body is a dental restoration, the dental body is typically composed of the glass-ceramic body. The dental restoration may be, but is not limited to, a crown, a partial crown, an abutment, an abutment crown, an inlay, an onlay, a veneer, a shell, or a multi-unit framework, or a bridge (e.g., 2-, 3- or 4- unit bridge) an implant bridge, and the like. The dental restoration may be colored. The dental restoration may have a color that matches a shade of by the VITA classical A1-D4^{®} shade guide with VITA Bleached Shades manufactured by Vita Zahnfabrik. The shade may be, but is not limited to, A1, A2, A3.5, A4, B1, B2, B3, B4, C1, C2, C3, C4, D1, D2, D3, D4, BL1 or BL2. The dental restoration may comprise a glazing.

In one embodiment, the dental body is a (colored) dental restoration having a multilayer structure or a gradient structure, and preferably a multilayer structure, as described herein, e.g., in section III.1.

In one embodiment, the dental body is a dental restoration comprising a (colored) glass-ceramic body comprising (or being composed of):
a top layer as described herein above, e.g., in section III.1, as an incisal or occlusal zone or a part thereof;
optionally at least one intermediate layer as described herein above, e.g., in section III.1, as a transition zone or a part thereof;
a bottom layer as described herein above, e.g., in section III.1, as a dentin zone or a part thereof.

In one embodiment, the dental body is a dental restoration comprising a (colored) glass-ceramic body comprising (or being composed of):
a top section as described herein above, e.g., in section III.1, as an incisal or occlusal zone or a part thereof;
an intermediate section as described herein above, e.g., in section III.1, as a transition zone or a part thereof;
a bottom section as described herein above, e.g., in section III.1, for preparing as a dentin zone or a part thereof.

### 3. Process for preparation

In one aspect, the present invention provides a process for preparing a dental body according to the present invention. The process steps vary depending on the internal structure and the type of the dental body.

### 3.1 Dental blank

In one embodiment, the process is a process for preparing a dental blank, like a dental mill blank or dental press blank. In one preferred embodiment, the process is a process for preparing a dental mill blank.

The process comprises the steps of:
- providing one or more powders selected from glass powders, glass-ceramic powders and mixtures thereof;
- preparing a shaped body from the one or more powders; and
- subjecting the shaped body to a heat treatment,
   or
- preparing a solid glass body from a molten glass; and
- subjecting the solid glass body to a heat treatment.

The process for preparing the dental blank depends on the internal structure of its glass-ceramic body.

### 3.1.1 Substantially homogenous internal structure

The dental blank may comprise a glass-ceramic body having a substantially homogenous internal structure.

The process for preparing the dental blank may comprise the steps of:
- providing a powder selected from glass powders, glass-ceramic powders and mixtures thereof;
- preparing a shaped body from the powder; and
- subjecting the shaped body to a heat treatment.

The step of providing the powder, the step of preparing the shaped body, and the step of heat treating the shaped may be as described herein above, e.g., in section II.4.1.1, II.4.1.2, and II.4.1.3, respectively.

The shaped (molded) body typically has a pre-shape of the glass-ceramic body of the dental blank. "Pre-shape" of a dental blank as used herein means that the shaped body has already a desired shape of a dental blank to be made from the pre-shaped body but in enlarged dimensions. The shaped (molded) body with the pre-shape of the dental blank can be densified to the final shape of the dental blank in the heat treating step.

The heat treating step provides the glass-ceramic body of the dental blank. The process may comprise one or more additional process steps which are typical in the art, and which may be carried out before, between or after the process steps described herein. Additional steps may be, but are not limited to, adjusting the particle size distribution of the powder (e.g., by sieving), preparing the surface of the glass-ceramic body (e.g., by grinding, lapping or polishing), providing the glass-ceramic body with one or more outside layers, like protective layers or printing layers (e.g., containing product information, a bar code, or a QR code), and/or providing the glass-ceramic body with a holding pin.

In an alternative embodiment, the process for preparing the dental mill blank may comprise the steps of:
- preparing a solid glass body from a molten glass; and
- subjecting the solid glass body to a heat treatment.

The step of providing the solid glass body and the step of heat treating the solid glass body may be as described herein above, e.g., in section II.4.2, and particularly the process described in section II.4.2 which comprises a single heat treatment.

The solid glass body typically has the shape of the glass-ceramic body of the dental blank. The heat treating step provides the glass-ceramic body of the dental blank. The process may comprise one or more additional process steps which are typical in the art, and which may be carried out before, between or after the process steps described herein. Additional steps may be, but are not limited to, preparing the surface of the glass-ceramic body (e.g., by grinding, lapping or polishing), providing the glass-ceramic body with one or more outside layers, like protective layers or printing layers (e.g., containing product information, a bar code, or a QR code), and/or providing the glass-ceramic body with a holding pin.

### 3.2.1 Multilayer structure or gradient structure

The dental blank may comprise a glass-ceramic body having a multilayer structure or a gradient structure.

The process for preparing the dental blank may comprise the steps of:
- providing two or more powders selected from glass powders, glass-ceramic powders and mixtures thereof;
- preparing a shaped body from the powders; and
- subjecting the shaped body to a heat treatment.

The process comprises the step of providing two or more powders selected from glass-powders, glass-ceramic powders and mixtures thereof. The number of the two or more powders are not particularly limited and may depend, e.g., on the number of layers in the glass-ceramic body. The powders differ in their chemical composition. At least one of the powders is a glass powder or glass-ceramic powder according to the present invention, and particularly a glass powder according to the present invention. One of the powders may be a different glass powder or glass-ceramic powder which is suitable for forming a glass-ceramic but which does not form a keatite s.s. crystal phase when subjected to the heat treating step. This different glass or glass-ceramic powder may be used for preparing a layer or a section of the dental blank which does not contain a keatite s.s. crystal phase.

In one specific embodiment, the two or more powders are a glass powder or glass-ceramic powder according to the present invention, and particularly glass powders according to the present invention. Each one of the two or more (glass) powders may be provided as described herein, e.g., in section II.4.1.1. Typically, each one of the two or more powders are provided in form of granules additionally comprising one or more binders and one or more pigments. The one or more pigments typically differ for each type of granules so that different layers or sections of the dental blank exhibit a different color.

The process further comprises the step of preparing a shaped body from the powders. The shaped body may be porous. The shaped body may be prepared by molding the powders in a suitable mold to obtain a molded body. The molded body typically has a pre-shape of a dental blank. The pre-shape may have the desired form of the dental blank. The preparation of the molded body may differ depending on the form in which the powders are provided. When the powders are provided in dry form, e.g., in form of a dry blend with one or more additives, the powders may be introduced into the mold followed by a compacting step (e.g., a pressing step, like an uniaxial pressing). The compacting step may be a cold compacting step. The cold compacting (e.g., cold pressing) may be carried out at a temperature of less than 60°C, like in a range of 15 to 35°C, and at a pressure of 2 to 30 bar, like in a range of 5 to 15 bar. Additionally or alternatively, the compacting step may be a hot compacting step. The hot compacting step (e.g., hot pressing step) may be carried out at a temperature in a range of 650 to 850°C, like in the range of 700 to 800°C, and a pressure in a range of 5 to 50 MPa, preferably 10 to 30 MPa. The hot compacting is typically carried out over a time period in a range of 0.1 to 10 min, like in a range of 0.3 to 5 min. The hot compacting step is preferably carried at an atmospheric pressure below ambient pressure, for example, at an atmospheric pressure of less than 0.1 bar, like in a range of 0.01 to 0.8 bar. The hot compacting may be a pressure sintering. After the hot compacting, a compacted body may be obtained which is essentially non-porous or which does not contain a significant porosity.

When the powders are provided in liquid form, the powders may be introduced into the mold followed by removing the liquid. The mold may be a mold suitable for die casting or slip casting. The mold may have pores through which the liquid can be removed. Removal of the liquid may be carried out and/or supported by, e.g., pressing, suction, and/or freeze drying.

The internal structure of the shaped body may differ depending on the desired internal structure of the dental blank, like a multilayer structure or a gradient structure.

When it is intended that the dental blank has a multilayer structure, the shaped body is typically prepared such that it comprises different powder layers.

The shaped body may comprise (or may be composed of):
a top powder layer,
optionally at least one intermediate powder layer (e.g., 1 to 6 intermediate powder layers, 1 to 4 intermediate powder layers, 2 intermediate powder layers, or 1 intermediate powder layer), and
a bottom powder layer.

The powder layers are prepared such that a weight ratio of the two or more powders (e.g., two or more glass powders) differs in each one of the powder layers.

For example, when the shaped body is prepared from two powders according to the present invention (i.e., a first powder and a second powder), the powder layers are prepared such that a weight ratio of the two powders differs in each one of the powder layers. The powder layers may be prepared such that a weight ratio of the first powder to the second powder decreases in a direction from the top powder layer to the bottom powder layer. This is to be understood in that a weight content of the first powder decreases in a direction from the top powder layer to the bottom powder layer, and a weight content of the second powder increases in a direction from the bottom powder layer to the top powder layer. This includes that the second powder may essentially be absent (i.e., about 0 wt.%) from the top powder layer and/or the first powder may essentially be absent (i.e., about 0 wt.%) from the bottom powder layer.

It is also possible that each powder layer is prepared from a different type of powder according to the present invention. Furthermore, it is possible that the top powder layer and the optional at least one intermediate powder layer are prepared from different types of powders according to the present invention, and the bottom layer is prepared from a powder which is not suitable for forming a keatite s.s. crystal phase during the heat treating step.

In certain embodiments, the shaped body comprises (or is composed of)
a top powder layer, wherein the top powder layer is composed of a first powder, optionally in combination with one or more additives (e.g., one or more binders and one or more pigments),
optionally at least one intermediate powder layer, wherein the at least one intermediate powder layer is composed of a mixture of the first powder and a second powder, each one optionally in combination with one or more additives (e.g., one or more binders and one or more pigments),
a bottom powder layer, wherein the bottom powder layer is composed of the second powder, optionally in combination with one or more additives (e.g., one or more binders and one or more pigments).

In certain other embodiments, the shaped body comprises (or is composed of)
a top powder layer, wherein the top powder layer is composed of a first powder (e.g., the second glass powder), optionally in combination with one or more additives (e.g., one or more binders and one or more pigments),
optionally at least one intermediate powder layer, wherein each one of the at least one intermediate powder layer is composed of a different powder, each one optionally in combination with one or more additives (e.g., one or more binders and one or more pigments),
a bottom powder layer, wherein the bottom powder layer is composed of a second powder, optionally in combination with one or more additives (e.g., one or more binders and one or more pigments).

When it is intended that the dental blank has a gradient structure, the shaped body is typically prepared such that it comprises an intermediate powder section which has a gradually changing weight amount of its bulk powders.

Thus, in an alternative embodiments, the molded body comprises (or is composed of):
a top powder section,
an intermediate powder section, and
a bottom powder section.

The top powder section is prepared from a powder according to the present invention and the bottom section is prepared from a different type of powder according to the present invention or from a powder which is not suitable for forming a keatite s.s. crystal phase during the heat treating step. The weight amount of the powder of the top powder section gradually changes across the intermediate powder section in a direction of the bottom powder section. Such a gradual change can be achieved by using a deposition device or dosing device known in the art. Such a deposition device or a dosing device can be adjusted to continuously change a weight content of components in a mixture that is added into a mold.

The process further comprises the step of subjecting the shaped body to a heat treatment. The heat treatment provides the glass-ceramic body of the dental blank. The heat treating step may be as described herein above, e.g. in section II.4.3.1.

The process may comprise one or more additional process steps which are typical in the art, and which may be carried out before, between or after the process steps described herein. Additional steps may be, but are not limited to, adjusting the particle size distribution of the powder (e.g., by sieving), preparing the surface of the glass-ceramic body (e.g., by grinding, lapping or polishing), providing the glass-ceramic body with one or more outside layers, like protective layers or printing layers (e.g., containing product information, a bar code, or a QR code), and/or providing the glass-ceramic body with a holding pin.

According to one embodiment, the process for preparing the dental blank comprises the steps of:
- providing two or more different types of glass powders according to the present invention, and wherein each one of the two or more glass powder are provided in form of granules at least comprising the respective glass powder, one or more binders, and one or more pigments,
- preparing a shaped body from the two or more glass powders, wherein the shaped body comprises
   a top powder layer,
   optionally at least one intermediate powder layer (e.g., 1 to 6 intermediate powder layers, 1 to 4 intermediate powder layers, 2 intermediate powder layers, or 1 intermediate powder layer), and
   a bottom powder layer,
   wherein a weight amount of the two or more glass powders differs in each one of the powder layers of the shaped body, and
- heat treating the shaped body to obtain the glass-ceramic body of the dental blank.

### 3.2 Dental restoration

In one embodiment, the process is a process for preparing a dental restoration.

The dental restoration may be prepared from a dental blank, preferably a dental mill blank, according to the present invention. In particular, the process for preparing the dental restoration may comprise the steps of:
- providing a dental mill blank according to the present invention (e.g., as described herein above in section III.3.1);
- machining the dental mill blank to provide a dental restoration; and
- optionally surface-treating the dental restoration.

Alternatively, the dental restoration may be prepared by a process comprising the steps of:
- providing a solid glass body, preferably a solid glass body as described herein in section I.2;
- subjecting the solid glass body to a first heat treatment for providing a dental mill blank which is composed of a solid glass-ceramic body comprising a lithium metasilicate crystal phase (but which is essentially free of a keatite s.s. crystal phase);
- machining the dental mill blank according to provide a precursor of a dental restoration; and
- subjecting the precursor of the dental restoration to a second heat treatment to provide a dental restoration which is composed of a glass-ceramic body comprising the lithium alumosilicate glass-ceramic according to the present invention; and
- optionally surface-treating the dental restoration.

The machining of the dental mill blank may be carried out by any conventional process for machining a dental mill blank, and typically by a CAD/CAM process. Such processes are known in the art. The machining may include, but is not limited to, cutting, drilling and/or grinding the dental mill blank. The dental restoration may optionally be subjected to a surface-treating as is known in the art, e.g., by polishing, coloring and/or glazing the surface of the dental restoration.

Alternatively, the dental restoration may be prepared by an additive manufacturing process, and preferably by a stereo lithography process. In particular, the process for preparing the dental restoration may comprise the steps of:
- providing one or more suspensions comprising a glass powder and/or glass-ceramic powder according to the present invention, an organic monomer solution, and a photo initiator;
- depositing and curing the one or more suspensions on a solid platform to provide a pre-cursor of a dental restoration; and
- subjecting the pre-cursor of the dental restoration to a hear treatment to provide a dental restoration.

The step of depositing and curing may be carried out in that a layer of the one or more suspensions is deposited on the solid platform followed by curing a part of the deposited layer by selective light exposure, e.g., by a light source irradiating light having a wavelength of less than 1000 nm, and preferably UV light. The curing takes place by (radical) polymerization of the organic monomers initiated by the photo initiator. The step of depositing and curing of a layer is carried out as often as needed for providing the desired pre-cursor.

The heat treating may be a heat treating step as described herein, e.g., in section II.4.3.1. The precursor of the dental restoration typically comprises a substantial amount of an organic component, like an organic polymer, obtained in the curing step. Therefore, the heat treating typically comprises a heating step for debinding the pre-cursor of the dental restoration.

In the following, the present invention will be further described by selected examples. The examples provided herein are not to be construed as limiting the present invention in any way.

### IV. Examples

### 1. Measuring methods

### 1.1 Crystal phases

Crystal phases were determined by X-ray diffraction (XRD) analysis. The XRD measurements were carried out in Bragg-Brentano geometry on a high resolution Bruker D8-Advance powder diffractometer with Cu-Kalpha1 radiation from a Ge(111) Johannson primary monochromator. On the secondary side, the powder diffraction pattern was recorded by a position sensitive LynxEye Silicon-Strip detector (Bruker AXS) with 3.5° aperture angle. The measurement time per sample was 16 hours for the range of 5 to 110° and a step size of 0.0118° 2 theta.

Weight amounts of the crystal phases were determined by quantitative Rietveld analysis. The quantitative Rietveld analysis was performed using the PDF5 database (ICDD) and Topas software (Bruker AXS).

### 1.2 Fracture toughness (K_{1C})

The fracture toughness (K_{1C}) was determined according to the Single Edge V-Notched Beam (SEVNB) method according to DIN EN ISO 6872, and particularly DIN EN ISO 6872:2015.

### 1.3 Color: contrast ratio (opacity) and CIE L*a*b* color space

The contrast ratio (opacity) was determined according to BS 5612, and particularly BS 5612:1978. The contrast ratio was determined using a test body having a thickness of 2.0 mm. CIE L*a*b* color space was measured according to DIN 6174 using a test body having a thickness of 2.0 mm. Colors (CIE L*, a*, b*) were measured against a background of: L* = 93.1; a* = (-0.64); b* = 4.22. The measurements were made using a spectrophotometer CM 3700-D (Konica-Minolta). Prior to the measurement, the test bodies were wet ground to a thickness of 2.0 mm (± 0.02 mm) using a rotating diamond grinding disc and 1000 SiC grinding paper, followed by a final surface wet polishing using a diamond grind disc (20 µm).

### 1.4 Coefficient of thermal expansion (CTE)

The coefficient of thermal expansion (CTE) was determined using a dilatometer according to DIN EN ISO 6872, and particularly DIN EN ISO 6872:2015. The measurements were carried out over a temperature range of from 100 to 500°C.

### 1.5 Glass transition temperature Tg

The glass transition temperature Tg was determined according to ISO 11357-2. The measurement was carried out using the device STA449 Jupiter F3 which is commercially available by the company Netzsch.

### 2. Examples 1 to 67 (powder route)

### 2.1 Preparation

Examples 1 to 67 describe lithium alumosilicate glass-ceramic dental bodies which were prepared from a lithium alumino silicate glass powder.

To obtain the desired glass powder, the individual raw materials for the glass powder were weighted, combined and homogenized in a laboratory speed mixer. The individual raw materials were selected to obtain a glass with a chemical composition as described in tables I-A to I-I. For example, the glass powder for preparing the lithium alumosilicate glass-ceramic dental body according to example 1 were prepared from the following raw materials: silicon oxide, lithium carbonate, sodium carbonate, potassium carbonate, aluminum metaphosphate, aluminumoxyhydroxy hydrate and zirconium oxide.

Subsequently, the raw materials were melted at a temperature Tₛ for a time period tₛ as indicated in tables I-A to I-I. The melt was casted in water to obtain a glass. The glass was dried at 150 °C for about 1 hour in an oven and then ground in a laboratory mill to obtain a glass powder. A powder fraction having a particle size of <45 µm was separated and used for preparing a molded body of the glass powder.

The glass powder was introduced into a mold and flattened with a stamp (only stamp weight without additional external pressure). Afterwards, the glass powder was pressed with a pressure of 10 bar to obtain a molded powder body. The molded body was removed from the mold by turning the mold 180° and pushing the molded body out of the mold by applying a pressure in the opposite direction.

The molded powder body was then fired under vacuum in a sintering furnace (Programat P500) using a heating rate of 10 K/min. The sintering was carried out at a temperature Tₛᵢₙₜₑᵣ for a time period tₛᵢₙₜₑᵣ as indicated in tables I-A to I-I. Optionally, the heating steps included a nucleation step at a temperature T_{nuc} for a time period of t_{nuc} as shown in tables I-A to I-I.

### 2.2 Composition and processing parameters

**Table I-A**

| **Example No.** | **1** | **2** | **3** | **4** | **5** | **6** | **7** | **8** |
|---|---|---|---|---|---|---|---|---|
| *Oxide (wt%)* | | | | | | | | |
| SiO₂ | 64.70 | 65.89 | 72.83 | 72.20 | 71.20 | 70.20 | 69.20 | 68.20 |
| Li₂O | 12.00 | 12.23 | 15.74 | 8.50 | 8.50 | 8.50 | 8.50 | 8.50 |
| Na₂O | 1.30 | 0.00 | 0.00 | 1.30 | 1.30 | 1.30 | 1.30 | 1.30 |
| K₂O | 2.10 | 1.60 | 0.00 | 1.50 | 1.50 | 1.50 | 1.50 | 1.50 |
| MgO | 0.00 | 0.00 | 3.83 | 0.00 | 0.00 | 0.00 | 0.00 | 0.00 |
| Al₂O₃ | 9.90 | 10.08 | 3.52 | 10.50 | 10.50 | 11.50 | 12.50 | 13.50 |
| ZrO₂ | 4.00 | 4.08 | 0.58 | 4.00 | 4.00 | 4.00 | 4.00 | 4.00 |
| P₂O₅ | 6.00 | 6.12 | 3.50 | 2.00 | 3.00 | 3.00 | 3.00 | 3.00 |
| **Σ** | 100.00 | 100.00 | 100.00 | 100.00 | 100.00 | 100.00 | 100.00 | 100.00 |
| | | | | | | | | |
| Mol (SiO₂:Li₂O) | 2.68 | 2.35 | 2.30 | 4.22 | 4.17 | 4.10 | 4.05 | 3.99 |
| Wt. (Li₂O:Al₂O₃) | 1.21 | 1.21 | 4.47 | 0.81 | 0.81 | 0.74 | 0.68 | 0.63 |
| Σ 1-valent ions | 15.4 | 13.8 | 15.7 | 11.3 | 11.3 | 11.3 | 11.3 | 11.3 |
| Σ 1-valent ions (w/o Li₂O) | 3.4 | 1.6 | 0.0 | 2.8 | 2.8 | 2.8 | 2.8 | 2.8 |
| Σ 2-valent ions | 0.0 | 0.0 | 3.8 | 0.0 | 0.0 | 0.0 | 0.0 | 0.0 |
| Σ 3-valent ions | 9.9 | 10.1 | 3.5 | 10.5 | 10.5 | 11.5 | 12.5 | 13.5 |
| Σ 4-valent ions | 4.0 | 4.1 | 0.6 | 4.0 | 4.0 | 4.0 | 4.0 | 4.0 |
| Σ 5-valent ions | 6.0 | 6.1 | 3.5 | 2.0 | 3.0 | 3.0 | 3.0 | 3.0 |
| | | | | | | | | |
| T_{g} [°C] glass frit | 494 | 508 | 458 | 520 | 525 | 525 | 528 | 534 |
| Ts [°C] | 1550 | 1500 | 1500 | 1600 | 1600 | 1600 | 1600 | 1600 |
| ts [min] | 60 | 60 | 60 | 60 | 60 | 60 | 60 | 60 |
| Tnuc / °C | 600 | | | 600 | 600 | 600 | 600 | 600 |
| t_{nuc} [min] | 30 | | | 30 | 30 | 30 | 30 | 30 |
| Tsinter [°C] | 880 | 840 | 920 | 880 | 880 | 880 | 880 | 880 |
| tsinter [min] | 30 | 30 | 30 | 30 | 30 | 30 | 30 | 30 |

**Table I-B**

| **Example No.** | **9** | **10** | **11** | **12** | **13** | **14** | **15** | **16** |
|---|---|---|---|---|---|---|---|---|
| *Oxide (wt%)* | | | | | | | | |
| SiO₂ | 67.20 | 64.70 | 64.70 | 64.70 | 64.70 | 64.70 | 65.00 | 64.00 |
| Li₂O | 8.50 | 12.00 | 12.00 | 12.00 | 12.00 | 12.00 | 14.00 | 13.00 |
| Na₂O | 1.30 | 1.30 | 1.30 | 1.30 | 1.30 | 1.30 | 1.30 | 1.30 |
| K₂O | 1.50 | 2.10 | 2.10 | 2.10 | 2.10 | 2.10 | 2.10 | 2.10 |
| Al₂O₃ | 14.50 | 9.40 | 8.90 | 9.90 | 9.90 | 9.90 | 9.90 | 9.90 |
| ZrO₂ | 4.00 | 4.00 | 4.00 | 4.50 | 0.00 | 0.00 | 4.70 | 4.70 |
| Ta₂O₅ | 0.00 | 0.00 | 0.00 | 0.00 | 4.90 | 0.00 | 0.00 | 0.00 |
| Nb₂O₅ | 0.00 | 0.00 | 0.00 | 0.00 | 0.00 | 4.90 | 0.00 | 0.00 |
| P₂O₅ | 3.00 | 6.50 | 7.00 | 5.50 | 5.10 | 5.10 | 3.00 | 5.00 |
| **Σ** | 100.00 | 100.00 | 100.00 | 100.00 | 100.00 | 100.00 | 100.00 | 100.00 |
| | | | | | | | | |
| Mol (SiO₂:Li₂O) | 3.93 | 2.68 | 2.68 | 2.68 | 2.68 | 2.68 | 2.31 | 2.45 |
| Wt. (Li₂O:Al₂O₃) | 0.59 | 1.28 | 1.35 | 1.21 | 1.21 | 1.21 | 1.41 | 1.31 |
| Σ 1-valent ions | 11.3 | 15.4 | 15.4 | 15.4 | 15.4 | 15.4 | 17.4 | 16.4 |
| Σ 1-valent ions (w/o Li₂O) | 2.8 | 3.4 | 3.4 | 3.4 | 3.4 | 3.4 | 3.4 | 3.4 |
| Σ 2-valent ions | 0.0 | 0.0 | 0.0 | 0.0 | 0.0 | 0.0 | 0.0 | 0.0 |
| Σ 3-valent ions | 14.5 | 9.4 | 8.9 | 9.9 | 9.9 | 9.9 | 9.9 | 9.9 |
| Σ 4-valent ions | 4.0 | 4.0 | 4.0 | 4.5 | 0.0 | 0.0 | 4.7 | 4.7 |
| Σ 5-valent ions | 3.0 | 6.5 | 7.0 | 5.5 | 10.0 | 10.0 | 3.0 | 5.0 |
| | | | | | | | | |
| T_{g} [°C] glass frit | 538 | 499 | 501 | 498 | 484 | 490 | 476 | 491 |
| Ts [°C] | 1600 | 1550 | 1550 | 1550 | 1550 | 1550 | 1550 | 1550 |
| ts [min] | 60 | 60 | 60 | 60 | 60 | 60 | 60 | 60 |
| Tnuc / °C | 600 | 600 | 600 | 600 | 600 | 600 | 600 | 600 |
| t_{nuc} [min] | 30 | 30 | 30 | 30 | 30 | 30 | 30 | 30 |
| Tsinter [°C] | 880 | 880 | 880 | 880 | 880 | 880 | 880 | 870 |
| tsinter [min] | 30 | 30 | 30 | 30 | 30 | 30 | 30 | 30 |

**Table I-C**

| **Example No.** | **17** | **18** | **19** | **20** | **21** | **22** | **23** | **24** |
|---|---|---|---|---|---|---|---|---|
| *Oxide (wt%)* | | | | | | | | |
| SiO₂ | 63.31 | 64.00 | 63.00 | 72.20 | 73.15 | 72.06 | 69.01 | 70.41 |
| Li₂O | 14.87 | 13.00 | 14.80 | 9.90 | 15.15 | 14.93 | 14.30 | 14.59 |
| Na₂O | 1.31 | 3.00 | 3.00 | 1.30 | 0.00 | 0.00 | 0.00 | 0.00 |
| K₂O | 2.21 | 0.50 | 0.50 | 1.50 | 0.00 | 0.00 | 0.00 | 0.00 |
| MgO | 0.00 | 0.00 | 0.50 | 0.00 | 3.80 | 0.00 | 0.00 | 0.00 |
| CaO | 0.00 | 0.00 | 0.00 | 0.00 | 0.00 | 5.21 | 0.00 | 0.00 |
| SrO | 0.00 | 0.00 | 0.00 | 0.00 | 0.00 | 0.00 | 9.22 | 0.00 |
| ZnO | 0.00 | 0.00 | 0.00 | 0.00 | 0.00 | 0.00 | 0.00 | 7.39 |
| Al₂O₃ | 9.55 | 9.80 | 9.50 | 10.50 | 3.84 | 3.79 | 3.63 | 3.71 |
| ZrO₂ | 3.72 | 4.70 | 3.70 | 2.60 | 0.58 | 0.58 | 0.55 | 0.55 |
| P₂O₅ | 5.03 | 5.00 | 5.00 | 2.00 | 3.48 | 3.43 | 3.29 | 3.35 |
| **Σ** | 100.00 | 100.00 | 100.00 | 100.00 | 100.00 | 100.00 | 100.00 | 100.00 |
| | | | | | | | | |
| Mol (SiO₂:Li₂O) | 2.12 | 2.45 | 2.12 | 3.63 | 2.40 | 2.40 | 2.40 | 2.40 |
| Wt. (Li₂O:Al₂O₃) | 1.56 | 1.33 | 1.56 | 0.94 | 3.95 | 3.94 | 3.94 | 3.93 |
| Σ 1-valent ions | 18.4 | 16.5 | 18.3 | 12.7 | 15.2 | 14.9 | 14.3 | 14.6 |
| Σ 1-valent ions (w/o Li₂O) | 3.5 | 3.5 | 3.5 | 2.8 | 0.0 | 0.0 | 0.0 | 0.0 |
| Σ 2-valent ions | 0.0 | 0.0 | 0.5 | 0.0 | 3.8 | 5.2 | 9.2 | 7.4 |
| Σ 3-valent ions | 9.6 | 9.8 | 9.5 | 10.5 | 3.8 | 3.8 | 3.6 | 3.7 |
| Σ 4-valent ions | 3.7 | 4.7 | 3.7 | 2.6 | 0.6 | 0.6 | 0.6 | 0.6 |
| Σ 5-valent ions | 5.0 | 5.0 | 5.0 | 2.0 | 3.5 | 3.4 | 3.3 | 3.4 |
| | | | | | | | | |
| T_{g} [°C] glass frit | 480 | 485 | 466 | 494 | 461 | 468 | 461 | 461 |
| Ts [°C] | 1550 | 1550 | 1550 | 1600 | 1500 | 1500 | 1500 | 1500 |
| ts [min] | 60 | 60 | 60 | 60 | 60 | 60 | 60 | 60 |
| Tnuc / °C | 600 | 600 | 600 | 600 | 540 | 560 | 540 | 540 |
| t_{nuc} [min] | 30 | 30 | 30 | 30 | 30 | 30 | 30 | 30 |
| Tsinter [°C] | 870 | 870 | 870 | 880 | 900 | 930 | 920 | 940 |
| tsinter [min] | 30 | 30 | 30 | 30 | 10 | 10 | 10 | 10 |

**Table I-D**

| **Example No.** | **25** | **26** | **27** | **28** | **29** | **30** | **31** | **32** |
|---|---|---|---|---|---|---|---|---|
| *Oxide (wt%)* | | | | | | | | |
| SiO₂ | 72.27 | 71.16 | 69.01 | 70.41 | 74.42 | 74.58 | 70.50 | 68.32 |
| Li₂O | 14.97 | 14.74 | 14.30 | 14.59 | 10.58 | 15.45 | 14.61 | 14.15 |
| MgO | 3.75 | 0.00 | 0.00 | 0.00 | 0.00 | 2.28 | 3.66 | 3.55 |
| CaO | 0.00 | 5.14 | 0.00 | 0.00 | 0.00 | 0.00 | 0.00 | 0.00 |
| SrO | 0.00 | 0.00 | 4.61 | 0.00 | 0.00 | 0.00 | 0.00 | 0.00 |
| ZnO | 0.00 | 0.00 | 0.00 | 3.69 | 3.69 | 0.00 | 0.00 | 0.00 |
| Al₂O₃ | 5.00 | 5.00 | 8.24 | 7.41 | 7.41 | 3.56 | 4.88 | 4.73 |
| ZrO₂ | 0.57 | 0.57 | 0.55 | 0.55 | 0.55 | 0.59 | 3.00 | 6.00 |
| P₂O₅ | 3.44 | 3.39 | 3.29 | 3.35 | 3.35 | 3.54 | 3.35 | 3.25 |
| **Σ** | 100.00 | 100.00 | 100.00 | 100.00 | 100.00 | 100.00 | 100.00 | 100.00 |
| | | | | | | | | |
| Mol (SiO₂:Li₂O) | 2.40 | 2.40 | 2.40 | 2.40 | 3.50 | 2.40 | 2.40 | 2.40 |
| Wt. (Li₂O:Al₂O₃) | 2.99 | 2.95 | 1.74 | 1.97 | 1.43 | 4.34 | 2.99 | 2.99 |
| Σ 1-valent ions | 15.0 | 14.7 | 14.3 | 14.6 | 10.6 | 15.5 | 14.6 | 14.2 |
| Σ 1-valent ions (w/o Li₂O) | 0.0 | 0.0 | 0.0 | 0.0 | 0.0 | 0.0 | 0.0 | 0.0 |
| Σ 2-valent ions | 3.8 | 5.1 | 4.6 | 3.7 | 3.7 | 2.3 | 3.7 | 3.6 |
| Σ 3-valent ions | 5.0 | 5.0 | 8.2 | 7.4 | 7.4 | 3.6 | 4.9 | 4.7 |
| Σ 4-valent ions | 0.6 | 0.6 | 0.6 | 0.6 | 0.6 | 0.6 | 3.0 | 6.0 |
| Σ 5-valent ions | 3.4 | 3.4 | 3.3 | 3.4 | 3.4 | 3.5 | 3.4 | 3.3 |
| | | | | | | | | |
| T_{g} [°C] glass frit | 466 | 468 | 469 | 465 | 488 | 462 | 474 | 486 |
| Ts [°C] | 1500 | 1500 | 1500 | 1500 | 1500 | 1500 | 1500 | 1500 |
| ts [min] | 60 | 60 | 60 | 60 | 60 | 60 | 60 | 60 |
| Tnuc / °C | 540 | 540 | 540 | 540 | 530 | | 540 | 540 |
| t_{nuc} [min] | 30 | 30 | 30 | 30 | 30 | | 30 | 30 |
| Tsinter [°C] | 900 | 910 | 940 | 940 | 960 | 920 | 920 | 870 |
| tsinter [min] | 30 | 30 | 30 | 30 | 60 | 30 | 30 | 30 |

**Table I-E**

| **Example No.** | **33** | **34** | **35** | **36** | **37** | **38** | **39** | **40** |
|---|---|---|---|---|---|---|---|---|
| *Oxide (wt%)* | | | | | | | | |
| SiO₂ | 69.96 | 65.65 | 76.66 | 70.50 | 68.67 | 70.10 | 66.99 | 67.98 |
| Li₂O | 14.50 | 13.60 | 9.53 | 14.61 | 14.23 | 14.52 | 14.30 | 14.08 |
| MgO | 0.00 | 0.00 | 0.00 | 3.66 | 0.00 | 3.64 | 0.00 | 0.00 |
| CaO | 5.06 | 0.00 | 0.00 | 0.00 | 0.00 | 0.00 | 4.99 | 0.00 |
| SrO | 0.00 | 4.39 | 0.00 | 0.00 | 0.00 | 0.00 | 0.00 | 0.00 |
| ZnO | 0.00 | 0.00 | 3.74 | 0.00 | 3.60 | 0.00 | 0.00 | 3.56 |
| Al₂O₃ | 4.92 | 7.84 | 7.51 | 4.88 | 7.23 | 4.85 | 4.85 | 7.15 |
| La₂O₃ | 0.00 | 0.00 | 0.00 | 0.00 | 0.00 | 0.00 | 3.00 | 0.00 |
| Y₂O₃ | 0.00 | 0.00 | 0.00 | 0.00 | 0.00 | 3.00 | 0.00 | 0.00 |
| Gd₂O₃ | 0.00 | 0.00 | 0.00 | 0.00 | 0.00 | 0.00 | 0.00 | 2.00 |
| Dy₂O₃ | 0.00 | 0.00 | 0.00 | 0.00 | 0.00 | 0.00 | 0.00 | 2.00 |
| ZrO₂ | 0.56 | 0.52 | 0.56 | 0.00 | 0.00 | 0.56 | 0.56 | 0.00 |
| GeO₂ | 0.00 | 0.00 | 0.00 | 0.00 | 0.00 | 0.00 | 2.02 | 0.00 |
| Ta₂O₅ | 0.00 | 0.00 | 0.00 | 3.00 | 0.00 | 0.00 | 0.00 | 0.00 |
| Nb₂O₅ | 0.00 | 0.00 | 0.00 | 0.00 | 3.00 | 0.00 | 0.00 | 0.00 |
| P₂O₅ | 5.00 | 8.00 | 2.00 | 3.35 | 3.27 | 3.33 | 3.29 | 3.23 |
| **Σ** | 100.00 | 100.00 | 100.00 | 100.00 | 100.00 | 100.00 | 100.00 | 100.00 |
| | | | | | | | | |
| Mol (SiO₂:Li₂O) | 2.40 | 2.40 | 4.00 | 2.40 | 2.40 | 2.40 | 2.33 | 2.40 |
| Wt. (Li₂O:Al₂O₃) | 2.95 | 1.73 | 1.27 | 2.99 | 1.97 | 2.99 | 2.95 | 1.97 |
| Σ 1-valent ions | 14.5 | 13.6 | 9.5 | 14.6 | 14.2 | 14.5 | 14.3 | 14.1 |
| Σ 1-valent ions (w/o Li₂O) | 0.0 | 0.0 | 0.0 | 0.0 | 0.0 | 0.0 | 0.0 | 0.0 |
| Σ 2-valent ions | 5.1 | 4.4 | 3.7 | 3.7 | 3.6 | 3.6 | 5.0 | 3.6 |
| Σ 3-valent ions | 4.9 | 7.8 | 7.5 | 4.9 | 7.2 | 7.9 | 7.9 | 11.2 |
| Σ 4-valent ions | 0.6 | 0.5 | 0.6 | 0.0 | 0.0 | 0.6 | 2.6 | 0.0 |
| Σ 5-valent ions | 5.0 | 8.0 | 2.0 | 6.4 | 6.3 | 3.3 | 3.3 | 3.2 |
| | | | | | | | | |
| T_{g} [°C] glass frit | 466 | 486 | 495 | 472 | 475 | 475 | 466 | 472 |
| Ts [°C] | 1500 | 1500 | 1500 | 1500 | 1500 | 1500 | 1500 | 1500 |
| ts [min] | 60 | 60 | 60 | 60 | 60 | 60 | 60 | 60 |
| Tnuc / °C | 540 | 520 | 520 | 500 | 510 | 520 | 530 | 520 |
| t_{nuc} [min] | 30 | 30 | 30 | 30 | 30 | 30 | 30 | 30 |
| Tₛᵢₙₜₑᵣ [°C] | 930 | 980 | 960 | 920 | 940 | 900 | 920 | 940 |
| tsinter [min] | 30 | 90 | 60 | 60 | 60 | 60 | 60 | 60 |

**Table I-F**

| **Example No.** | **41** | **42** | **43** | **44** | **45** | **46** | **47** | **48** |
|---|---|---|---|---|---|---|---|---|
| *Oxide (wt%)* | | | | | | | | |
| SiO₂ | 67.60 | 69.66 | 66.27 | 65.55 | 73.95 | 69.65 | 66.07 | 62.37 |
| Li₂O | 14.19 | 14.74 | 12.29 | 12.17 | 14.71 | 17.32 | 20.53 | 23.86 |
| Na₂O | 0.00 | 0.00 | 1.06 | 2.12 | 0.00 | 0.00 | 0.00 | 0.00 |
| MgO | 0.00 | 0.00 | 0.00 | 0.00 | 3.80 | 3.83 | 3.94 | 4.05 |
| CaO | 0.00 | 5.14 | 0.00 | 0.00 | 0.00 | 0.00 | 0.00 | 0.00 |
| ZnO | 3.54 | 0.00 | 0.00 | 0.00 | 0.00 | 0.00 | 0.00 | 0.00 |
| Al₂O₃ | 7.11 | 5.00 | 10.13 | 10.02 | 3.49 | 5.11 | 5.25 | 5.40 |
| Eu₂O₃ | 0.00 | 1.00 | 0.00 | 0.00 | 0.00 | 0.00 | 0.00 | 0.00 |
| ZrO₂ | 0.00 | 0.57 | 4.10 | 4.06 | 0.58 | 0.59 | 0.61 | 0.62 |
| SnO₂ | 1.32 | 0.00 | 0.00 | 0.00 | 0.00 | 0.00 | 0.00 | 0.00 |
| CeO₂ | 3.02 | 0.00 | 0.00 | 0.00 | 0.00 | 0.00 | 0.00 | 0.00 |
| P₂O₅ | 3.22 | 3.39 | 6.15 | 6.08 | 3.47 | 3.50 | 3.60 | 3.70 |
| F | 0.00 | 0.50 | 0.00 | 0.00 | 0.00 | 0.00 | 0.00 | 0.00 |
| **Σ** | 100.00 | 100.00 | 100.00 | 100.00 | 100.00 | 100.00 | 100.00 | 100.00 |
| | | | | | | | | |
| Mol (SiO₂:Li₂O) | 2.37 | 2.35 | 2.35 | 2.35 | 2.50 | 2.00 | 1.60 | 1.30 |
| Wt. (Li₂O:Al₂O₃) | 2.00 | 2.95 | 1.21 | 1.21 | 4.21 | 3.39 | 3.91 | 4.42 |
| Σ 1-valent ions | 14.2 | 14.7 | 13.4 | 14.3 | 14.7 | 17.3 | 20.5 | 23.9 |
| Σ 1-valent ions (w/o Li₂O) | 0.0 | 0.0 | 1.1 | 2.1 | 0.0 | 0.0 | 0.0 | 0.0 |
| Σ 2-valent ions | 3.5 | 5.1 | 0.0 | 0.0 | 3.8 | 3.8 | 3.9 | 4.1 |
| Σ 3-valent ions | 7.1 | 6.0 | 10.1 | 10.0 | 3.5 | 5.1 | 5.3 | 5.4 |
| Σ 4-valent ions | 4.3 | 0.6 | 4.1 | 4.1 | 0.6 | 0.6 | 0.6 | 0.6 |
| Σ 5-valent ions | 3.2 | 3.4 | 6.2 | 6.1 | 3.5 | 3.5 | 3.6 | 3.7 |
| | | | | | | | | |
| T_{g} [°C] glass frit | 471 | 453 | 508 | 499 | 459 | 455 | 443 | 436 |
| Ts [°C] | 1500 | 1500 | 1500 | 1500 | 1500 | 1500 | 1500 | 1500 |
| ts [min] | 60 | 60 | 60 | 60 | 60 | 60 | 60 | 60 |
| Tnuc / °C | 520 | 520 | | | | | | |
| t_{nuc} [min] | 30 | 30 | | | | | | |
| Tsinter [°C] | 950 | 920 | 850 | 850 | 920 | 890 | 890 | 920 |
| tsinter [min] | 60 | 60 | 30 | 30 | 30 | 30 | 30 | 30 |

**Table I-G**

| **Example No.** | **49** | **50** | **51** | **52** | **53** | **54** | **55** | **56** |
|---|---|---|---|---|---|---|---|---|
| *Oxide (wt%)* | | | | | | | | |
| SiO₂ | 74.83 | 76.40 | 77.66 | 72.55 | 72.83 | 73.11 | 73.40 | 73.70 |
| Li₂O | 13.29 | 11.87 | 10.73 | 15.03 | 15.09 | 15.15 | 15.21 | 15.27 |
| MgO | 3.73 | 3.68 | 3.64 | 3.77 | 3.79 | 3.80 | 3.81 | 3.83 |
| Al₂O₃ | 4.19 | 4.14 | 4.10 | 4.63 | 4.26 | 3.88 | 3.51 | 3.13 |
| ZrO₂ | 0.57 | 0.56 | 0.56 | 0.58 | 0.58 | 0.58 | 0.58 | 0.58 |
| P₂O₅ | 3.39 | 3.35 | 3.31 | 3.44 | 3.45 | 3.48 | 3.49 | 3.49 |
| Σ | 100.00 | 100.00 | 100.00 | 100.00 | 100.00 | 100.00 | 100.00 | 100.00 |
| | | | | | | | | |
| Mol (SiO₂:Li₂O) | 2.80 | 3.20 | 3.60 | 2.40 | 2.40 | 2.40 | 2.40 | 2.40 |
| Wt. (Li₂O:Al₂O₃) | 3.17 | 2.87 | 2.62 | 3.25 | 3.54 | 3.90 | 4.33 | 4.88 |
| Σ 1-valent ions | 13.3 | 11.9 | 10.7 | 15.0 | 15.1 | 15.2 | 15.2 | 15.3 |
| Σ 1-valent ions (w/o Li₂O) | 0.0 | 0.0 | 0.0 | 0.0 | 0.0 | 0.0 | 0.0 | 0.0 |
| Σ 2-valent ions | 3.7 | 3.7 | 3.6 | 3.8 | 3.8 | 3.8 | 3.8 | 3.8 |
| Σ 3-valent ions | 4.2 | 4.1 | 4.1 | 4.6 | 4.3 | 3.9 | 3.5 | 3.1 |
| Σ 4-valent ions | 0.6 | 0.6 | 0.6 | 0.6 | 0.6 | 0.6 | 0.6 | 0.6 |
| Σ 5-valent ions | 3.4 | 3.4 | 3.3 | 3.4 | 3.5 | 3.5 | 3.5 | 3.5 |
| | | | | | | | | |
| T_{g} [°C] glass frit | 467 | 473 | 474 | 458 | 460 | 458 | 458 | 461 |
| Ts [°C] | 1500 | 1500 | 1500 | 1500 | 1500 | 1500 | 1500 | 1500 |
| ts [min] | 60 | 60 | 60 | 60 | 60 | 60 | 60 | 60 |
| Tnuc / °C | | | | | | | | |
| t_{nuc} [min] | | | | | | | | |
| Tₛᵢₙₜₑᵣ [°C] | 900 | 900 | 900 | 920 | 920 | 920 | 920 | 920 |
| tsinter [min] | 30 | 30 | 30 | 30 | 30 | 30 | 30 | 30 |

**Table I-H**

| **Example No.** | **57** | **58** | **59** | **60** | **61** | **62** | **63** | **64** |
|---|---|---|---|---|---|---|---|---|
| *Oxide (wt%)* | | | | | | | | |
| SiO₂ | 73.98 | 73.00 | 72.60 | 72.22 | 73.61 | 74.11 | 74.59 | 67.40 |
| Li₂O | 15.33 | 15.13 | 15.05 | 14.97 | 15.25 | 15.35 | 15.46 | 13.41 |
| MgO | 3.84 | 3.79 | 3.77 | 3.75 | 3.83 | 3.85 | 3.88 | 0.00 |
| SrO | 0.00 | 0.00 | 0.00 | 0.00 | 0.00 | 0.00 | 0.00 | 4.47 |
| Al₂O₃ | 2.74 | 3.49 | 3.47 | 3.45 | 3.51 | 3.14 | 2.77 | 9.50 |
| ZrO₂ | 0.58 | 0.58 | 0.58 | 0.57 | 0.58 | 0.59 | 0.59 | 0.53 |
| P₂O₅ | 3.53 | 4.01 | 4.53 | 5.04 | 3.22 | 2.96 | 2.71 | 4.69 |
| Σ | 100.00 | 100.00 | 100.00 | 100.00 | 100.00 | 100.00 | 100.00 | 100.00 |
| | | | | | | | | |
| Mol (SiO₂:Li₂O) | 2.40 | 2.40 | 2.40 | 2.40 | 2.40 | 2.40 | 2.40 | 2.50 |
| Wt. (Li₂O:Al₂O₃) | 5.59 | 4.34 | 4.34 | 4.34 | 4.34 | 4.89 | 5.58 | 1.41 |
| Σ 1-valent ions | 15.3 | 15.1 | 15.1 | 15.0 | 15.3 | 15.4 | 15.5 | 13.4 |
| Σ 1-valent ions (w/o Li₂O) | 0.0 | 0.0 | 0.0 | 0.0 | 0.0 | 0.0 | 0.0 | 0.0 |
| Σ 2-valent ions | 3.8 | 3.8 | 3.8 | 3.8 | 3.8 | 3.9 | 3.9 | 4.5 |
| Σ 3-valent ions | 2.7 | 3.5 | 3.5 | 3.5 | 3.5 | 3.1 | 2.8 | 9.5 |
| Σ 4-valent ions | 0.6 | 0.6 | 0.6 | 0.6 | 0.6 | 0.6 | 0.6 | 0.5 |
| Σ 5-valent ions | 3.5 | 4.0 | 4.5 | 5.0 | 3.2 | 3.0 | 2.7 | 4.7 |
| | | | | | | | | |
| T_{g} [°C] glass frit | 457 | 460 | 464 | 462 | 463 | 459 | 455 | 473 |
| Ts [°C] | 1500 | 1500 | 1500 | 1500 | 1500 | 1500 | 1500 | 1500 |
| tₛ [min] | 60 | 60 | 60 | 60 | 60 | 60 | 60 | 60 |
| T_{nuc} / °C | | | | | | | | |
| t_{nuc} [min] | | | | | | | | |
| Tₛᵢₙₜₑᵣ [°C] | 920 | 920 | 920 | 920 | 930 | 930 | 930 | 950 |
| tsinter [min] | 30 | 30 | 30 | 30 | 30 | 30 | 30 | 30 |

**Table I-I**

| **Example No.** | **65** | **66** | **67** |
|---|---|---|---|
| *Oxide (wt%)* | | | |
| SiO₂ | 67.68 | 65.66 | 68.40 |
| Li₂O | 14.96 | 14.20 | 13.41 |
| MgO | 3.56 | 0.00 | 0.00 |
| SrO | 0.00 | 0.00 | 4.47 |
| ZnO | 0.00 | 3.47 | 0.00 |
| Al₂O₃ | 6.00 | 7.50 | 5.50 |
| ZrO₂ | 2.54 | 3.52 | 2.53 |
| P₂O₅ | 5.26 | 5.65 | 5.69 |
| Σ | 100.00 | 100.00 | 100.00 |
| | | | |
| Mol (SiO₂:Li₂O) | 2.25 | 2.30 | 2.54 |
| Wt. (Li₂O:Al₂O₃) | 2.49 | 1.89 | 2.44 |
| Σ 1-valent ions | 15.0 | 14.2 | 13.4 |
| Σ 1-valent ions (w/o Li₂O) | 0.0 | 0.0 | 0.0 |
| Σ 2-valent ions | 3.6 | 3.5 | 4.5 |
| Σ 3-valent ions | 6.0 | 7.5 | 5.5 |
| Σ 4-valent ions | 2.5 | 3.5 | 2.5 |
| Σ 5-valent ions | 5.3 | 5.7 | 5.7 |
| | | | |
| T_{g} [°C] glass frit | 467 | 476 | 477 |
| Ts [°C] | 1500 | 1500 | 1500 |
| ts [min] | 60 | 60 | 60 |
| Tnuc / °C | | | |
| t_{nuc} [min] | | | |
| Tₛᵢₙₜₑᵣ [°C] | 920 | 930 | 930 |
| tsinter [min] | 30 | 30 | 30 |

### 2.3 Crystal phases and physical properties

The obtained lithium alumosilicate glass-ceramic dental bodies contain crystal phases as provided in tables II-A to II-C. Selected examples were tested for physical properties as provided in table III-A (fracture toughness), table III-B (opacity and colors), and table III-C (coefficient of thermal expansion).

**Table II-A**

| **Example No.** | **1** | **2** | **3** | **4** | **5** | **6** |
|---|---|---|---|---|---|---|
| Crystal phases | Keatite s.s. Li₂Si₂O₅ Li₃PO₄ | Keatite s.s. Petalite quartz s.s. Li₂Si₂O₅ Li₂SiO₃ Li₃PO₄ | Li₂Si₂O₅ Keatite s.s. quartz s.s. Li₃PO₄ | quartz s.s. Keatite s.s. Li₂Si₂O₅ Li₃PO₄ | Keatite s.s. quartz s.s. Li₂Si₂O₅ Li₃PO₄ | Keatite s.s. Li₃PO₄ |

| **Example No.** | **7** | **8** | **9** | **10** | **11** | **12** |
|---|---|---|---|---|---|---|
| Crystal phases | Keatite s.s. Li₃PO₄ | Keatite s.s. Li₃PO₄ | Keatite s.s. (43.8 wt.%) Li₃PO₄ (4.8 wt.%) Li₂SiO₃ (1.4 wt.%) | Keatite s.s. quartz s.s. Li₂Si₂O₅ Li₃PO₄ | quartz s.s. Keatite s.s. Li₂Si₂O₅ Li₃PO₄ | Keatite s.s. Li₂Si₂O₅ Li₃PO₄ |

| **Example No.** | **13** | **14** | **15** | **16** | **17** | **18** |
|---|---|---|---|---|---|---|
| Crystal Phases | Keatite s.s. (25.7 wt.%) Li₂Si₂O₅ (25.4 wt.%) Li₃PO₄ (10.6 wt.%) | Keatite s.s. Li₂Si₂O₅ Li₃PO₄ | Keatite s.s. quartz s.s. Li₂SiO₃ Li₃PO₄ | Keatite s.s. quartz s.s. Li₂Si₂O₅ Li₂SiO₃ Li₃PO₄ | quartz s.s. Li₂SiO₃ Keatit s.s. Li₂Si₂O₅ Li₃PO₄ | Keatit s.s. Li₂Si₂O₅ Li₂SiO₃ Li₃PO₄ quartz s.s. |

| **Example No.** | **19** | **20** | **21** | **22** | **23** | **24** |
|---|---|---|---|---|---|---|
| Crystal phases | Keatite s.s. Li₂SiO₃ quartz s.s. Li₃PO₄ | Keatite s.s. Li₂Si₂O₅ Li₃PO₄ | Li₂Si₂O₅ Keatite s.s. quartz s.s. Li₃PO₄ | Li₂Si₂O₅ Keatite s.s. quartz s.s. Li₃PO₄ | Li₂Si₂O₅ Keatite s.s. LiSr(PO₄) quartz s.s. Li₃PO₄ | Li₂Si₂O₅ quartz s.s. Keatite s.s. Li₂ZnSiO₄ Li₃PO₄ |

**Table II-B**

| **Example No.** | **25** | **26** | **27** | **28** | **29** | **30** |
|---|---|---|---|---|---|---|
| Crystal phases | Li₂Si₂O₅ Keatite s.s. quartz s.s. Li₃PO₄ | Li₂Si₂O₅ Keatite s.s. Li₃PO₄ «Li₂SiO₃ | Keatite s.s. Li₂Si₂O₅ Li₃PO₄ | Keatite s.s. Li₂Si₂O₅ Li₂ZnSiO₄ LiZnPO₄ Li₃PO₄ | quartz s.s. Keatite s.s. Li₂SiO₃ Li₃PO₄ Li₂ZnSiO₄ | Li₂Si₂O₅ Keatite s.s. quartz s.s. Li₃PO₄ |

| **Example No.** | **31** | **32** | **33** | **34** | **35** | **36** |
|---|---|---|---|---|---|---|
| Crystal phases | Li₂Si₂O₅ Keatite s.s. Li₃PO₄ | Li₂Si₂O₅ Keatite s.s. quartz s.s. Li₃PO₄ | Li₂Si₂O₅ Keatite Li₃PO₄ quartz s.s. | Keatite s.s. Li₂SiO₃ Li₃PO₄ | quartz s.s. Keatite s.s. Li₂SiO₃ Li₃PO₄ Li₂ZnSiO₄ | Li₂Si₂O₅ Keatite s.s. Li₃PO₄ |

| **Example No.** | **37** | **38** | **39** | **40** | **41** | **42** |
|---|---|---|---|---|---|---|
| Crystal Phases | Keatite s.s. Li₂Si₂O₅ Li₃PO₄ Li₂Zn(SiO₄) Li₄Zn(PO₄)₂ | Li₂Si₂O₅ Keatite s.s. Li₃PO₄ | Li₂Si₂O₅ Keatite s.s. Li₃PO₄ | Keatite s.s. Li₂Si₂O₅ Li₃PO₄ Li₂Zn(SiO₄) | Keatite s.s. Li₂Si₂O₅ CeO₂ Li₃PO₄ Li₂Zn(SiO₄) | Li₂Si₂O₅ Keatite s.s. Li₃PO₄ Ca₅(PO₄)₃F |

| **Example No.** | **43** | **44** | **45** | **46** | **47** | **48** |
|---|---|---|---|---|---|---|
| Crystal phases | Keatite s.s. Li₂Si₂O₅ quartz s.s. Li₂SiO₃ Li₃PO₄ | Keatite s.s. Li₂Si₂O₅ quartz s.s. Li₂SiO₃ Li₃PO₄ | Li₂Si₂O₅ Keatite s.s. quartz s.s. Li₃PO₄ | Li₂Si₂O₅ Keatite s.s. Li₂SiO₃ Li₃PO₄ | Li₂SiO₃ Keatit s.s. Li₃PO₄ | Li₂SiO₃ (47.1 wt.%) Keatit s.s. (18.5 wt. %) Li₃PO₄ (9.6 wt.%) |

**Table II-C**

| **Example No.** | **49** | **50** | **51** | **52** | **53** | **54** |
|---|---|---|---|---|---|---|
| Crystal phases | Li₂Si₂O₅ Li₃PO₄ Keatite s.s. quartz s.s. | quartz s.s. Li₂Si₂O₅ Keatite s.s. Li₃PO₄ | quartz s.s. Li₂Si₂O₅ Keatite s.s. Li₃PO₄ | Li₂Si₂O₅ Keatite s.s. Li₃PO₄ | Li₂Si₂O₅ Keatite s.s. Li₃PO₄ | Li₂Si₂O₅ Li₃PO₄ Keatite s.s. quartz s.s. |

| **Example No.** | **55** | **56** | **57** | **58** | **59** | **60** |
|---|---|---|---|---|---|---|
| Crystal phases | Li₂Si₂O₅ Li₃PO₄ Keatite s.s. quartz s.s. | Li₂Si₂O₅ Li₃PO₄ Keatite s.s. quartz s.s. | Li₂Si₂O₅ Li₃PO₄ Keatite s.s. quartz s.s. | Li₂Si₂O₅ (48.2 wt.%) Keatite s.s. (15.2 wt.%) Li₃PO₄ (7.4 wt.%) quartz s.s. (4.9 wt.%) | Li₂Si₂O₅ Li₃PO₄ Keatite s.s. quartz s.s. | Li₂Si₂O₅ Li₃PO₄ Keatite s.s. quartz s.s. |

| **Example No.** | **61** | **62** | **63** | **64** | **65** | **66** |
|---|---|---|---|---|---|---|
| Crystal Phases | Li₂Si₂O₅ Li₃PO₄ Keatite s.s. quartz s.s. | Li₂Si₂O₅ Li₃PO₄ Keatite s.s. quartz s.s. | Li₂Si₂O₅ Li₃PO₄ Keatite s.s. quartz s.s. | Keatite s.s. Li₂Si₂O₅ Li₃PO₄ | Keatite s.s. Li₂Si₂O₅ Li₃PO₄ | Keatite s.s. Li₂Si₂O₅ Li₃PO₄ |

| **Example No.** | **67** | | | | | |
|---|---|---|---|---|---|---|
| Crystal phases | Keatite s.s. Li₂Si₂O₅ quartz s.s. Li₃PO₄ | | | | | |

**Table III-A**

| **Example No.** | **3** | **45** | **58** | **59** | **60** |
|---|---|---|---|---|---|
| Fracture toughness K_{1C} [MPa*m^{0.5}] | 2.70 | 2.60 | 2.58 | 2.55 | 2.55 |
| SEVNB Method | | | | | |

**Table III-B**

| **Example No.** | **6** | **7** | **8** | **9** | **31** | **32** | **33** |
|---|---|---|---|---|---|---|---|
| Opacity | 59.09 | 59.46 | 59.90 | 66.37 | 90.29 | 99.56 | 96.09 |
| L* | 89.99 | 89.82 | 89.94 | 86.40 | 90.06 | 93.93 | 92.55 |
| a* | -0.11 | -0.06 | -0.03 | 0.22 | 0.47 | 0.18 | 0.25 |
| b* | 5.85 | 5.91 | 5.82 | 7.13 | 5.17 | 1.56 | 3.96 |

| **Example No.** | **52** | **53** | **54** | **55** | **56** | **57** | **58** |
|---|---|---|---|---|---|---|---|
| Opacity | 94.91 | 93.90 | 92.85 | 85.60 | 87.38 | 88.27 | 76.44 |
| L* | 92.43 | 91.52 | 91.38 | 94.24 | 92.62 | 91.74 | 91.29 |
| a* | 0.33 | 0.39 | 0.25 | -0.24 | -0.01 | 0.09 | 0.03 |
| b* | 3.21 | 3.99 | 3.96 | 2.70 | 4.11 | 4.63 | 5.37 |

| **Example No.** | **59** | **60** | **62** | **63** | | | |
|---|---|---|---|---|---|---|---|
| Opacity | 76.61 | 69.07 | 87.38 | 88.27 | | | |
| L* | 90.56 | 89.75 | 92.62 | 91.74 | | | |
| a* | 0.12 | 0.13 | -0.01 | 0.09 | | | |
| b* | 5.97 | 6.80 | 4.11 | 4.63 | | | |

**Table III-C**

| **Example No.** | **6** | **7** | **8** | **9** | **31** | **32** | **33** |
|---|---|---|---|---|---|---|---|
| CTE [1*10⁻⁶*K⁻¹] (100-500°C) | 4.61 | 4.52 | 4.35 | 4.09 | 7.43 | 7.79 | 7.54 |

### 3. Examples 68 to 75 (solid glass route)

Examples 68 to 75 describe lithium alumosilicate glass-ceramic dental bodies which were prepared from a lithium alumino silicate solid glass.

The solid glasses were obtained as follows: the starting glass was melted in a batch size of about 100 to 200 grams of raw materials in a Pt/Rh10 crucible at 1500°C for 2h. After the first melting process the glasses were poured into water to obtain a glass frit. The glass frit was dried at 150°C for about 1 hour and remelted again to improve the homogeneighty of the glass. The glass melt was poured into graphite molds to get the desired block shape for CAD/CAM machining. The glass blocks were subsequently put into a furnace to release stresses and undergo a controlled nucleation.

The crystallization was carried out using a process with a two thermal (heat) treatments. The glass blocks were first heated up to 700°C and held at that temperature for 20 minutes to crystallize lithium metasilicate. Afterwards, the glass-ceramics were heated in a second heat treatment up to 850°C for 10 minutes to crystallize lithium disilicate and keatite s.s., and to obtain the final lithium alumosilicate glass-ceramic.

Alternatively, it is possible to carry out the crystallization in a single heat treatment in which the glass blocks are heated up by 10 K/min up to 850°C including a dwell time of 10 min to obtain the final lithium alumosilicate glass-ceramic comprising lithium disilicate, keatite s.s. and lithium orthophosphate crystal phases.

Tables IV-A and IV-B provide the processing parameters and the properties of the lithium alumosilicate glass-ceramic dental bodies according to examples 68 to 75. The first crystallization step was carried out at a temperature T_{K2} for a time period t_{K2} and the second crystallization step was carried out at a temperature T_{K1} for a time period t_{K1}. The heating included a nucleation step at a temperature T_{nuc} for a time period of t_{nuc}.

**Table IV-A**

| **Example No.** | **68** | **69** | **70** | **71** | **72** | **73** | **74** | **75** |
|---|---|---|---|---|---|---|---|---|
| Oxide (wt%) | | | | | | | | |
| SiO₂ | 69.45 | 68.34 | 69.32 | 68.25 | 69.09 | 68.89 | 68.40 | 68.89 |
| Li₂O | 15.35 | 15.11 | 15.32 | 15.08 | 15.26 | 15.22 | 15.11 | 15.22 |
| Na₂O | 0.51 | 0.70 | 0.70 | 0.70 | 0.70 | 0.71 | 0.70 | 0.71 |
| K₂O | 0.51 | 1.00 | 0.70 | 1.00 | 1.00 | 1.11 | 1.00 | 1.11 |
| MgO | 0.30 | 0.30 | 0.30 | 0.30 | 0.30 | 0.30 | 0.30 | 0.30 |
| ZnO | 1.02 | 1.00 | 1.00 | 1.00 | 1.00 | 1.01 | 1.00 | 1.01 |
| Al₂O₃ | 7.18 | 6.45 | 6.79 | 6.44 | 6.52 | 6.04 | 6.45 | 6.04 |
| Er₂O₃ | 0.00 | 0.10 | 0.00 | 0.10 | 0.00 | 0.00 | 0.10 | 0.00 |
| ZrO₂ | 1.21 | 1.20 | 1.20 | 1.20 | 1.20 | 1.21 | 1.20 | 1.21 |
| CeO₂ | 0.51 | 1.60 | 0.53 | 1.80 | 0.80 | 1.40 | 1.60 | 1.40 |
| V₂O₅ | 0.05 | 0.16 | 0.05 | 0.10 | 0.05 | 0.14 | 0.10 | 0.14 |
| P₂O₅ | 3.91 | 3.84 | 3.89 | 3.83 | 3.88 | 3.87 | 3.84 | 3.87 |
| Tb₄O₇ | 0.00 | 0.20 | 0.20 | 0.20 | 0.20 | 0.10 | 0.20 | 0.10 |
| **Σ** | 100.00 | 100.00 | 100.00 | 100.00 | 100.00 | 100.00 | 100.00 | 100.00 |
| | | | | | | | | |
| Mol (SiO₂:Li₂O) | 2.25 | 2.25 | 2.25 | 2.25 | 2.25 | 2.25 | 2.25 | 2.25 |
| Wt. (Li₂O:Al₂O₃) | 2.14 | 2.34 | 2.26 | 2.34 | 2.34 | 2.52 | 2.34 | 2.52 |
| Σ 1-valent ions | 16.4 | 16.8 | 16.7 | 16.8 | 17.0 | 17.0 | 16.8 | 17.0 |
| Σ 1-valent ions (w/o Li₂O) | 1.0 | 1.7 | 1.4 | 1.7 | 1.7 | 1.8 | 1.7 | 1.8 |
| Σ 2-valent ions | 1.3 | 1.3 | 1.3 | 1.3 | 1.3 | 1.3 | 1.3 | 1.3 |
| Σ 3-valent ions | 7.2 | 6.6 | 6.8 | 6.5 | 6.5 | 6.0 | 6.6 | 6.0 |
| Σ 4-valent ions | 1.7 | 2.8 | 1.7 | 3.0 | 2.0 | 2.6 | 2.8 | 2.6 |
| Σ 5-valent ions | 4.0 | 4.0 | 3.9 | 3.9 | 3.9 | 4.0 | 3.9 | 4.0 |
| | | | | | | | | |
| Tₛ [°C] | 1500 | 1500 | 1500 | 1500 | 1500 | 1500 | 1500 | 1500 |
| tₛ [min] | 120 | 120 | 120 | 120 | 120 | 120 | 120 | 120 |
| Tn [°C] | 500 | 500 | 500 | 500 | 500 | 500 | 500 | 500 |
| tn [min.] | 10 | 10 | 10 | 10 | 10 | 10 | 10 | 10 |
| T_{K2} [°C] | 700 | 700 | 700 | 700 | 700 | 700 | 700 | 700 |
| tK2 [min.] | 20 | 20 | 20 | 20 | 20 | 20 | 20 | 20 |
| T_{K1} [°C] | 850 | 850 | 850 | 850 | 850 | 850 | 850 | 850 |
| T_{K1} [min] | 10 | 10 | 10 | 10 | 10 | 10 | 10 | 10 |

**Table IV-B**

| **Example No.** | **68** | **69** | **70** | **71** |
|---|---|---|---|---|
| Crystal phases | Li₂Si₂O₅ Keatite s.s. Li₃PO₄ | Li₂Si₂O₅ Keatite s.s. Li₃PO₄ | Li₂Si₂O₅ Keatite s.s. Li₃PO₄ | Li₂Si₂O₅ Keatite s.s. Li₃PO₄ |
| CTE [1*10⁻⁶*K⁻¹] (100-500°C) | 8.21 | 8.60 | 8.62 | 8.62 |
| | | | | |

| **Example No.** | **72** | **73** | **74** | **75** |
|---|---|---|---|---|
| Crystal phases | Li₂Si₂O₅ Keatite s.s. Li₃PO₄ | Li₂Si₂O₅ Keatite s.s. Li₃PO₄ | Li₂Si₂O₅ Keatite s.s. Li₃PO₄ | Li₂Si₂O₅ Keatite s.s. Li₃PO₄ |
| CTE [1*10⁻⁶*K⁻¹] (100-500°C) | 8.69 | 8.75 | 8.92 | 9.01 |

## Claims

1. A glass or a glass-ceramic for preparing a lithium alumosilicate glass-ceramic comprising a keatite solid solution crystal phase, wherein the glass or the glass-ceramic comprises
60 to 78 wt.% of SiO₂,
8.5 to 24 wt.% of Li₂O,
2.0 to 15 wt.% of Al₂O₃, and
a nucleating agent.

2. The glass or glass-ceramic according to claim 1, wherein the glass or glass-ceramic is a powder.

3. A lithium alumosilicate glass-ceramic for a dental body, wherein the lithium alumosilicate glass-ceramic comprises
60 to 78 wt.% of SiO₂,
8.5 to 24 wt.% of Li₂O,
2.0 to 15 wt.% of Al₂O₃, and
a nucleating agent, and
wherein the lithium alumosilicate glass-ceramic comprises a keatite solid solution crystal phase.

4. The lithium alumosilicate glass-ceramic according to claim 3, wherein the molar ratio of SiO₂ to Li₂O is in a range of 1.50 to 4.22, preferably in a range of 1.80 to 4.00, and more preferably in a range of 2.00 to 3.80.

5. The lithium alumosilicate glass-ceramic according to claim 3 or 4, comprising the Al₂O₃ in a weight amount in a range of 2.0 to 10.0 wt.%.

6. The lithium alumosilicate glass-ceramic according to any one of claims 3 to 5, comprising the keatite solid solution crystal phase in an amount in a range of 2.5 to 50 wt.%, based on the total weight of the lithium alumosilicate glass-ceramic.

7. The lithium alumosilicate glass-ceramic according to any one of claims 3 to 6, wherein the keatite solid solution crystal phase is the main crystal phase of the lithium alumosilicate glass-ceramic,
and optionally wherein the lithium alumosilicate glass-ceramic comprises one or more of Li₃PO₄, Li₂Si₂O₅, quartz s.s., and Li₂SiO₃, as one or more minor crystal phases, optionally in combination with one or more additional minor crystal phases.

8. The lithium alumosilicate glass-ceramic according to any one of claims 3 to 6, wherein the main crystal phase of the lithium alumosilicate glass-ceramic is a quartz s. s., Li₂Si₂O₅ or Li₂SiO₃, and the keatite solid solution crystal phase is a minor crystal phase.

9. The lithium alumosilicate glass-ceramic according to claim 8, wherein the main crystal phase of the lithium alumosilicate glass-ceramic is Li₂Si₂O₅.

10. A process for preparing a lithium alumosilicate glass-ceramic according to any one of claims 3 to 9, the process comprising the steps of:
- providing a powder selected from glass powders, glass-ceramic powders and mixtures thereof;
- preparing a shaped body from the powder; and
- subjecting the shaped body to a heat treatment,
or
- preparing a solid glass body from a molten glass; and
- subjecting the solid glass body to a heat treatment.

11. A dental body comprising a glass-ceramic body comprising a lithium alumosilicate glass-ceramic according to any one of claims 3 to 9.

12. The dental body according to claim 11, wherein the glass-ceramic body comprises:
a top layer, and
a bottom layer,
wherein the top layer is a layer of one type of the lithium alumosilicate glass-ceramic, and the bottom layer is either a layer of a different type of the lithium alumosilicate glass-ceramic or a layer of a different glass-ceramic material not containing a keatite s.s. crystal phase, and
wherein the top layer has a weight amount of the keatite s.s. crystal phase which is higher than a weight amount of the keatite s.s. crystal phase in the bottom layer.

13. The dental body according to claim 12, wherein the glass-ceramic body further comprises at least one intermediate layer between the top layer and the bottom layer,
wherein the at least one intermediate layer is at least one layer of another different type of the lithium alumosilicate glass-ceramic, and
wherein the top layer, the at least one intermediate layer, and the bottom layer differ in their weight amount of a keatite s.s. crystal phase such that the weight amount of the keatite s.s. crystal phase increases from the bottom layer to the at least one intermediate layer, and from the at least one intermediate layer to the top layer.

14. The dental body according to any one of claims 10 to 13, wherein the glass-ceramic body is **characterized by** a translucency gradient and/or a gradient of a coefficient of thermal expansion.

15. The dental body according to any one of claims 10 to 14, wherein the dental body is a dental blank for preparing a dental restoration, or wherein the dental body is a dental restoration.
